# EUROPEAN PATENT SPECIFICATION

(11) **EP 1 849 486 B1**
(45) Date of publication and mention of the grant of the patent: **08.04.2015**
(21) Application number: 06712686.2
(22) Date of filing: 31.01.2006
(51) Int. Cl.: C08B 37/02, C08H 1/00, C08L 5/02, C08L 89/00, C09J 105/02, C09J 189/00, A61L 24/08, A61L 24/10

(54) **SELF-DEGRADABLE TWO-COMPONENT REACTIVE ADHESIVE FOR MEDICAL USE AND RESIN FOR MEDICAL USE**
SELBSTABBAUBARER REAKTIVER ZWEI-KOMPONENTEN-KLEBSTOFF ZUR MEDIZINISCHEN VERWENDUNG UND HARZ ZUR MEDIZINISCHEN VERWENDUNG
ADHESIF REACTIF AUTODEGRADABLE A DEUX COMPOSANTS DESTINE A UN USAGE MEDICAL ET RESINE DESTINEE A UN USAGE MEDICAL

(30) Priority: 31.01.2005 JP 2005054577; 29.03.2005 JP 2005128610
(43) Date of publication of application: 31.10.2007
(73) Proprietor: BMG INCORPORATED, Kyoto-shi, Kyoto 601-8023 (JP)
(72) Inventor: NAKAJIMA, Naoki, Kyoto 617-0002 (JP); HYON, Suong-Hyu, Kyoto 611-0021 (JP); KONDA, Masakazu, Makishima-cho Uji-shi, Kyoto, 6110041 (JP)
(74) Representative: Strehl Schübel-Hopf & Partner
(86) International application number: PCT/JP2006/301543
(87) International publication number: WO 2006/080523

(56) References cited:
- WO-A1-98/15299
- JP-A- 2000 503 883
- JP-A- 2005 021 454
- US-A- 5 583 114
- US-A1- 2005 002 893
- US-A1- 2005 002 893
- MO X. ET AL.: 'Kaishitsu Gelatin to Polysaccharide kara no Shiketsuzai, Gelatin to Tato o Mochiita Shiketsuzai no Kaihatsu. (Hemostatic Agent from Modified Gelatin and Polysaccharides)' POLYMER PREPRINTS, JAPAN vol. 48, no. 3, 1999, page 566, XP003001573

## Description

### Field of the Invention

This invention relates to a medical-use adhesive that is used for bonding of living tissues, filling or preventing of adhesion between living tissues, or for stopping of bleeding, after surgical operation or the like, as well as a medical-use resin. Especially, the invention relates to an adhesive that cures after mixing of first and second liquids and then decomposes to be fluidized after elapse of a certain period.

### Background of the Invention

As an adhesive for medical use, especially for surgical operation; (1) cyanoacrylate adhesives and (2) fibrin glue have been predominantly used.

### (1) Cyanoacrylate adhesive

The cyanoacrylate adhesive has been used in industrial use and household use, for original purpose of instantly adhering of; metal, plastics, rubber, wood, ceramics or the like. About ten varieties of the cyanoacrylate adhesive for medical use had been developed by year of 1968. This category of adhesive utilizes that; cyanoacrylate monomers are polymerized and cured in presence of a small amount of water that is a polymerization initiator. And, the polymerization and curing are rapid, and strength of adhesion on living tissues are high. However, cured resin has poor flexibility and is rigid and may thereby deter healing of wounds. Moreover, the cured resin is difficult to be decomposed as to be remained as a foreign body after being enveloped. Further, there has happened to be reported that formaldehyde is produced on way of decomposition of the resin as to exhibit cytotoxicity and/or injuring on tissue.

### (2) Fibrin glue

Fibrinogen forms fibrin masses by action of thrombin; such mechanism of coagulation of blood is utilized in this category of adhesive. The fibrin glue has high compatibility with living body and high convenience in usage and is widely used in arresting of bleeding from a suture part or the like after surgical operation and in enhancing of bonding and closure of tissues. The fibrin glue however has only poor level of adhesive strength so that produced fibrin masses may be occasionally peeled off from the tissue. Moreover, due to being a blood product, there remains a fear of virus transmission.

On the other hand, two-liquid adhesives for surgical use as in (3)-(6) below have been recently proposed.

### (3) Dextranaldehyde (oxidized dextran being a polyaldehyde)/high-molecular-weight chitosan WO 2003/035122 of AESCULAP AG & CO KG (DE); a counterpart of US2005/0002893 A1 and EP1438079 B1.

There has been proposed a two-liquid reaction adhesive for surgical use; a first liquid of which is 15wt% aqueous solution of dextranaldehyde; and a second liquid of which is 2wt% or 4wt% aqueous solution of high-molecular-weight chitosan (protasan^{™} UPCL213, FMC Biopolymers). In the adhesive, molar ratio of aldehyde groups to amino groups (aldehyde/amino molar ratio) is "at least three" according to a sole independent claim 1; and curing within 150 seconds was achieved by adopting the aldehyde/amino molar ratios of no less than 6, which are calculated from Table 2 and Table 1. Meanwhile, Table 3 and so on indicate that achieved shear strength was sufficient; and Table 8 indicates that more excellent adhesive strength was achieved by use of high-molecular-weight dextran having an average molecular weight no less than 400,000.

Moreover, last section ("4. Stoppage of Liver Bleeding") of the description indicates that; a two-part reactive adhesive having the aldehyde/amino molar ratio of 13.6 ("DA6" on Table 3), with first liquid to second liquid mixing ratio of 1/1, was effective as haemostatic sealant for rat liver. Curing time of this adhesive was about 15 seconds according to Table 2.

Tables 6 and 7 as well as explanations relevant to these tables indicates that sufficient adhesion strength was achieved even when 20wt% aqueous solution of polyvinylalcohol-vinylamine graft copolymer (PVALNH₂) is used in place of the chitosan aqueous solution. However, molecular weight and composition of the used copolymer were not specified. The curing time was also not specified.

### (4) Micelle-forming polymer with aldehyde end groups/high-molecular-weight polyallylamin JP-2005-021454A; NISHIDA, Hiroshi; YOKOYAMA, Masayuki, "Tissue adhesive formed of polymer micelle as effective component".

JP-2005-021454A discloses two-part reactive "adhesive for animal tissues". Its first liquid is aqueous micelle solution of a polymer having following structure; [end-aldehyde group]-[polyethylene glycol segment having molecular weight of 5500]-[polylactide segment having molecular weight of 4000]. Second liquid of the adhesive is aqueous solution of high-molecular-weight (no less than 60,000) polyallylamine.

It is indicated that; aqueous solution of poly-L-lysine (Table 3 as well as RUN 13-14 and 16 of Table 5) and chitosan aqueous solution (Table 4) may be used as the second liquid, in place of the polyallylamine solution. Paragraph 0031 indicates that oxidized starch and oxidized cellulose may be used for the first liquid. In respect of the poly-L-lysine, RUN 13-14 and 16 of Table 5 indicate that; high molecular weight of 700,000 is needed and a relatively low molecular weight of 30,000 is not adoptable. Moreover, Table 3 as well as RUN 12-16 of Table 5 indicates that; pH of the polylysine aqueous solution as the second liquid has to be no less than 9.0.

### (5) Starch aldehyde (oxidized starch being a polyaldehyde)/collagen WO98/15299; "ADHESIVE COMPOSITION WITH MACROMOLECULAR POLYALDEHYDE BASE AND METHOD FOR CROSS-LINKING COLLAGEN"; a counterpart of Japan's issued patent 323871.

As a first liquid, 0.5ml of 5wt% aqueous solution of solubilized starch that has been oxidized to form a polyaldehyde; please see first sentence of Example 3. Molecular weight of the solubilized starch "may be varied in a range of 10,000 to 200,000 dalton" as read from Example 1. While molecular weight of collagen is not mentioned in the specification, the molecular weight is thought to be about 300,000 as in typical collagens. The adhesive is described to be useful in bonding tissues of living body and to have ability for preventing adhesion among tissues. Please see Examples 3-6.

### (6) Gelatin/succinimidized poly-L-glutamate JP-9(1997)-103479 "Medical-use material and its production method".

Aqueous solution containing the gelatin, which is produced by heat-denaturing of collagen, is used as a first liquid; and, an aqueous solution containing succinimidized poly-L-glutamic acid is used as a second liquid; according to a disclosed medical-use adhesive.

Meanwhile, other than the two-part adhesives mentioned above, there have also been proposed medical-use adhesives as in (7)-(8) below.

### (7) Gelatin/dicarboxylic acid

Gelatin is reacted with dicarboxylic acid as to convert amino groups of the gelatin into carboxylic groups. Such adhesive is disclosed. Please see JP-11(1999)-239610A ("Medical-use material for bonding tissues of living body as well as production method thereof"). Medical-use adhesives based on the gelatin causes fear of BSE or the like due to use of risk portions of cattle, and hence are avoided to be used in clinical practice.

### (8) Urethane polymer

For example, there is disclosed an adhesive formed of an adduct that is produced by polyol and polyisocyanate. Please see JP2004-261590A ("Medical-use adhesive"). This adhesive has disadvantages in difficulty of handling due to high viscosity and in difficulty of adhesion at portions having lot of blood or body fluid.

### Disclosure of the Invention

### Problems to be Solved by the Invention

In regard of the fibrin glue, it is not able to induce modification or adjustment of physical property of the cured resin, such as improvement in flexibility in accordance with occasions arisen. Moreover, it is not able to induce prolongation of period, during which the cured resin is degraded in the living body, which lasts typically for one to three days. Meanwhile, cyanoacrylate resin requires a quite a long period for being completely degraded and absorbed in the living body; there is a report saying the period is longer than one year.

Thus, designing of the resin as to be completely degraded and absorbed within 1-2 weeks, for example, is not achievable in a practical sense.

For any of the above-mentioned adhesives, the cured resin does not exhibit that degradation or disintegration proceeds rapidly after lapse of a certain period. When period for keeping adhesion strength or the like is taken to be long enough, undesired resin layer or molded resin piece lasts for a considerably long time as to become a problem. Meanwhile, desired length of the period for keeping or timing of starting degradation differs in accordance with categories of diseases and types of surgery. Nevertheless, controlling of the timing of starting degradation in response to detailed requirements has been practically impossible.

When attempt is made to design a resin that rapidly disintegrates after elapse of a designed disintegration period, such designing undergoes considerable constraints as follows. Because of use in the living body, it is required to have high level of safety without cytotoxicity, tissue toxicity and carcinogenicity. Moreover, the adhesive resin have to satisfy properties required for medical-use adhesive in general, such as; (1) excellent adhesion performance on an adherent that is a water-containing living body; (2) relatively rapid curing and setting under normal temperature and normal pressure on surface of tissues of living body; and (3) flexibility at a level not to inhibit physical motion of the adherent while adhering on the adherent such as skins, vessels or organs until wound is healed.

The invention is made in view of the above and is aimed to provide a medical-use adhesive and a medical-use hydrogel resin; which disintegrates rapidly after elapse of a designed disintegration period and which is able to rather freely control or adjust the designed disintegration period, while satisfying properties required for medical-use adhesive in general.

### Means for solving the problems

Medical-use two-component reactive adhesive of the invention an adhesive according to claim 1.

Medical-use hydrogel resin of the invention is obtained by mixing; first liquid and second liquid according to claim 1 wherein reaction molar ratio of aldehyde groups to amino groups is in a range of 0.2 to 2.0; and when kept as hydrogel, the hydrogel resin changes to sol state by self-disintegration after elapse of a period for keeping hydrogel state, which may be freely set in a range of one day to one month.

### Advantageous effect of the invention

The resin of the invention disintegrates rapidly after elapse of designed disintegration period, which may be rather freely controlled or adjusted. The adhesive or resin does not have adverse effect such as cytotoxicity on living body and is excellent in adhesion strength on living tissues or the like. The hydrogel resin layer after the curing has flexibility. Time length requiring for the curing reaction may be controlled or adjusted by some extent, as to become a preferred extent.

### Best mode for carrying out the invention

The alpha-glucan aldhehyde comprising the first liquid is obtained by oxidizing the alpha-glucan as to introduce aldehyde groups, and has weight-average molecular weight in a range of 1000 to 200,000. The alpha-glucan denotes sugar chains that are formed by alpha linkage of glucoses with each other. Sugar residue of glucan, which is anhydro glucose unit, has a moleculer weight of 162.14. The alpha-glucan of the invention includes dextran, dextrin and pullulan, which may be used as mixed with the other. Starch or amylose may be used when properly degraded. Pullulan product of high molecular weight may also be used after being properly degraded. Introduction of the aldehyde groups is made by typical methods of periodate oxidation. For achieving proper extent of self-degradability, number of the aldehyde groups introduced in one anhydro glucose unit is preferrably 0.1 to 1.0, more preferrably 0.2 to 9.0, still more preferrably 0.3 to 0.8. For enhancing preservation stability of the first liquid, the preferred number of introduced aldehyde groups to one anhydro glucose unit is relatively low, and as low as 0.2 to 0.4 for example.

Among the alpha-glucan aldehyde, dextran aldehyde and dextrin aldehyde are especially preferred because of stable adhesion performance. Weight-average molecular weight of dextran, which is used in obtaining the dextran aldehyde, is preferrably 2000 to 200,000, and more preferably 2000 to 100,000. Adoptable are those commercially supplied by Pharmacosmos A/S for example; and are not only Dextran 40, Dextran 60 and Dextran 70 in medical-use grade but also Dextran T10 to Dextran T2000 among T-Dextran series. Meanwhile, dextrin product commercially supplied by Wako Pure Chemical Industries, Ltd in Japan may be used for example, in obtaining the dextrin aldehyde. Weight-average molecular weight of the dextrin is in a range of 1000 to 10,000 for example. Most preferred molecular weight of alpha-glucan aldehyde varies in accordance with detailed usage; and by selecting ones having certain molecular weight or molecular weight distribution, time length up to fluidization by self-disintegration may be adjusted. When molecular weight of the dextran aldehyde is too large, the fluidization by self-disintegration becomes excessively delayed. Meanwhile, when molecular weight of the dextran aldehyde is too small, time period for keeping the gel state becomes too short.

Weight-average molecular weight and molecular weight distribution of the alpha-glucan may be easily obtained by typical aqueous GPC (gel filteration chromatography, that is, size exclusion chromatography (SEC) in a formal naming) measurement. In detail, GPC columns formed of cross-linked water soluble polymer (TOSOH TSK gel G3000PW and G5000PW, with a TSK guard column PWH) are used as maintained at 40°C, and buffer solution(10mM KH₂PO₄+10mMK₂HPO₄) is used as eluant.

Amino-group-containing polymer for forming the second liquid is formed of a chain of amino-group-containing units, and has weight-average molecular weight in a range of 1000 to 20,000, preferrably in a range of 1000 to 10,000 and more preferrably in a range of 1500 to 8000. Preferably, the amino-group-conatining polymer does not include high-molecular weight fraction no less than 30,000.

Especially preferred amino-group-containing polymer is substantially consisting of a molecular fraction solely; in a range no less than 1000 and smaller than 30,000, preferably in a range of 1000 to 25,000, and more preferably in a range of 1000 to 20,000, when molecular weight is measured by SDS gel electrophoresis. The "substantially" means that molecular fraction less than 5% in weight ratio or such stain dot pattern should be neglected.

Molecular weight of the polylysine or other amino-group-containing polymer may be obtained with easiness and high accuracy, by any of following methods.

### (1) SDS-PAGE (sodium dodecyl sulfate-polyacrylamide gel electrophoresis)

Measurement is easily made by use of an electrophoresis apparatus and a densitograph (AE-6920V), of Atto Corporation in Japan.

### (2) Ion association chromatography

In ion association chromatography of high-performance liquid chromatography (HPLC), reverse-phase column (TSKgel ODS-80Ts) is used for measurement. In the mesurement, acetonitril is used as non-aqueous solvent while applying a gradient; and standard protein marker is used.

### (3) Aqueous GPC

Measurement may be made by use of above-mentioned aqueous GPC column for example, which is maintained at 40°C, and by use of an eluant (5% Ammonium Biphosphate/ 3% Acetonitril; pH = 4.0) that is formed by mixing of GPC-grade distilled water, phosphate buffer and acetonitril. For obtaining absolute molecular weight, low-angle laser light scattering technique may be used as combined with the GPC technique (GPC-LALLS).

For the amino-group-containing polymer used in the second liquid, preferred is epsilon-poly-L-lysine that is produced by use of microorganism or enzyme and has molecular weight in a range of 1000 to 20,000, especially in a range of 1000 to 6000. Alpha-poly-L-lysine may also be used. Chitosan oligomer or degraded chitosan may also be used when having the proper molecular weight and molecular-weight distribution. Polyglycerin or polyvinyl alcohol having been introduced with amino group side chains might also be used.

In detail epsilon-poly-L-lysine that is obtained by following may be used, for example. Adopted strain is Streptomyces albulus subsp. Lysinopolymerus that is shown in Japan's issued patent 3525190 or 3653766. Adopted culture medium is formed of; glucose 5wt%, yeast extract 0.5wt%, ammonium sulfate 1wt%, dipotassium hydrogen phosphate 0.08wt%, potassium dihydrogen phosphate 0.136wt%, magnesium sulfate heptahydrate 0.05wt%, zinc sulfate heptahydrate 0.004wt%, and ferrous sulfate heptahydrate 0.03wt%; and is adjusted to pH6.8. After cultivation in this culture medium, the epsilon-poly-L-lysine is separated as collected from the culture medium.

When the polylysine or other amino-group-containing polymer has a too large molecular weight or excessively includes a fraction too large in molecular weight; then time period up to the fluidization by the self-disintegration becomes excessively long.

The amino-group-containing polymer in a certain molecular weight range may be partly replaced with those having higher molecular weight or with those having lower molecular weight. For example, high-molecular-weight chitosan (molecular weight of 200,000 for example) may be added to polylysine solely consisting of molecular-weight fraction in a range of 1000 to 20,000, by up to about 1/1 weight ratio. Amino-group-introduced polyethylene glycol (PEG-NH₂), which is formed by introducing amino end groups into polyhydric polyethylene glycol having 2 to 8 hydroxyl groups and having molecular weight of 500 to 1000, may also be added. Preferred ones for such adding is those produced by using sucrose as starting compound as to be highly multifunctional.

Into the second liquid, acid or acidic salt is added as pH adjuster. Thus, pH of mixture of the first and second liquids at a time just mixed together is adjusted to a value in a range of 5.0 to 8.0, preferably in a range of 5.5 to 7.5, more preferably in a range of 6.5 to 7.5. The second liquid has a pH value preferably in a range of 7.0 to 9.0.

As the pH adjuster, preferably mono- or poly- carboxilic acid or anhydride thereof is added. Examples of preferred carboxilic acids are acetic acid, citric acid, succinic acid, glutaric acid, malic acid, fumaric acid and maleic acid, which are naturally occurred. These carboxilic acid have high pH adjusting ability due to buffering function and are harmless to living body. Inorganic acids or salts such as hydrochloric acid and sulfuric acid may be used alone or combined with the above-mentioned carboxylic acid, so long as the pH is adjusted to a proper value in a range of 5.0 to 8.0. Phosphate buffer solution may also be used.

Selecting either of mono-, di-, and tri- carboxylic acids as the pH adjuster enables controlling of time period up to fluidization of the cured gel, which is occurred by self-disintegration in a state of hydrogel. This is presumably due to a following reason. When polycarboxylic acid is used, quasi-cross linking is occurred between polymer chains of the polylysine or the amino-group-containing polymer, so as to delay the fluidization by the self-disintegration.

Molar ratio of aldehyde groups to amino groups, at a time the first and second liquids are mixed with each other, is no less than 0.1 and lower than 3, and is preferably in a range of 0.2 to 2.0, more preferably in a range of 0.5 to 1.5. The closer the aldehyde/amino molar ratio becomes to 1, the smaller the residue aldehyde groups or the residue amino groups becomes. Thus, this is meaningful in further decreasing toxicity.

Concentration of the alpha-glucan aldehyde in the first liquid is in a range of 5 to 50wt%, and preferably in a range of 15 to 25wt%. Concentration of the amino-groups-containing polymer in the second liquid is in a range of 0.5 to 60wt%, preferably in a range of 5 to 50wt% and more preferably in a range of 5 to 20wt%. Too low level of the concentration in the first or second liquid causes problems such as insufficient curing reaction; and too high level of the concentration induces high viscosity of the adhesive liquid as to make it difficult to be handled.

The first and second liquids may be easily sterilized by irradiation with radioactive rays, preferrably by irradiation with 10 to 50Kgy of electron beams and more preferrably by irradiation with 20 to 30Kgy of electron beams. Conditions of the irradiation sterilization is set in a manner that no adverse effect is incurred on performance of the adhesive such as cure time.

Mixing of the first and second liquids and application may be in various ways when to use the two-component reactive adhesive of the invention. For example, either of the first and second liquids is applied, as it is, on surface of the adherent, and subsequently another of the first and second liquids is applied on the adherent as to achieve the mixing. Alternatively, the first and second liquids are mixed in a mixing cavity within an application device and thereafter emitted from spray nozzle as to be applied as sprayed. For some instance, the adhesive may be used, other than in bonding, to obtain a gel resin in a sheet form or the like that is used to prevent adhesion between the tissues. Mixing ratio of the first liquid to the second liquid by volume is typically in a range of 0.5 to 2.0, preferably is set to be about 1.0, which means mixing with almost equal amounts.

When the first and second liquids are mixed with each other, aldehyde groups on the alpha-glucan aldehyde are reacted with amino groups on the amino-groups-containing polymer as to form Schiff linkages, which makes cross linkages so as to form hydrogel having network structure of the polymer chains. Resultantly, curing is made within 2 to 150 seconds, preferably within 3 to 100 seconds, and more preferably within 5 to 50 seconds, from the mixing. Preferred time period up to the curing from the mixing varies to a certain extent in accordance with usages, and is no less than 10 seconds, no less than 15 seconds in particular, when to penetrate into inside of living tissues to exhibit high level of adhesion strength.

Cured adhesive layer or resin in a state of hydrogel, which is formed by curing reaction, changes into fluid state by self-disintegration when a designed fluidization period has elapsed. In other words, the cured adhesive layer or the resin, if being kept as a hydrogel, changes into a fluidized state, which is a flowable sol state, even without undergoing enzymatic degradation. Therefore, the cured adhesive layer or the resin, when being placed in living body, disappears after elapse of a predetermined time period, by being absorbed or excreted. The designed disintegration time period may be arbitrarily set in a rage of a few hours to 4 months, typically in a range of one day to one month, especially in a range of two days to two weeks.

On contrary, conventional biodegradative resins, which are degraded and absorbed in living body only through enzymatic degradation, vary in respect of time period for the degradation. It has been difficult to design the conventional biodegradative resins so as to be rapidly degraded after elapse of a required time period for keeping the adhesion strength.

The time period for self-disintegration may be arbitrarily controlled and set; by selection or controlling of molecular weights of, and molecular-weight distribution, of the alpha-glucan aldehyde and/or the amino-group-containing polymer; by use or non-use and selection of polycarboxylic acids; and by controlling of pH at a time the first and second liquids are mixed. Thus, it is able to arbitrarily design the time period for being disintegrated and absorbed, by controlling of compositions of the two-component adhesive.

While mechanism of the self-disintegration is not clear, we consider that; alpha-glucoside linkage adjacent to the Schiff linkage has become easy to be cleaved where aldehyde groups of the alpha-glucan aldehyde have reacted with the amino groups.

Adhesives utilizing the Schiff base forming reaction have been conventionally used. Nevetheless, no adhesives having self-degradation property have been developed. Reason for this is presumably that; high-molecular-weight polymers have been used as the aldehyde-groups-containing polymer and as the amino-groups-containing polymer. In particular, as the amino-groups-containing polymers, ones having molecular weight no less than several dozen thousands have been used without exception. Because the amino-groups-containing polymer would not degrade, using of high-molecular-weight ones thereof leads to that; no disintegration of the cured resin occurs even when the alpha-aldehyde dextran is degraded. Meanwhile, even when molecular weight of the amino-groups-containing polymer is small, using of high-molecular-weight ones of the aldehyde-groups-containing polymer leads to that; cured resin would not be self-disintegrated within a month.

Conventionally, there has been presumably prevailed a prejudice that; high-molecular ones of the aldehyde-groups-containing polymer has to be used when for achieving the adhesion performance.

The medical-use adhesive and medical-use resin of the invention may be preferably applied for bonding in the living body, filling between tissues, hemostasis, embolization ov vessels, sealing of aneurysm, and substrate for drug delivery system (DDS).

### Embodiment

### 1. Controlling of amount of introducing aldehyde groups by sodium periodate.

Five grams of dextran (Wako Pure Chemical Industries, Ltd; Lot No.EWN0778) having weight-average molecular weight of 200,000 was dissolved in 100ml of distilled water. Then, various amount of sodium periodate (MW 213.89) was added and stirred at 40°C for 5 hours as to proceed reaction. After the reaction, solution was subjected to dialysis for 24 hours by use of distilled water and dialysis membrane of fractioning molecular weight of 14,000, and thereafter being freeze dried. Thus, the dextran aldehyde was obtained.

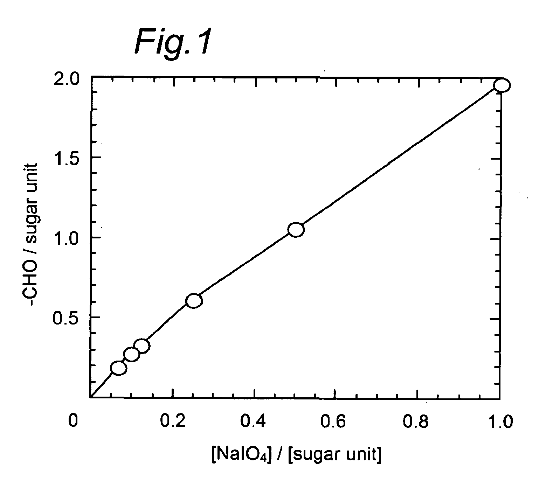

In respect of each dextran aldehyde product, amount of introduced aldehyde groups was measured. Obtained is a relationship between; molar amounts of the added sodium periodate and molar amounts of the introduced aldehyde groups, with respect to each anhydro glucose unit of dextran. Result is shown in Fig. 1.

The amount of the introduced aldehyde groups was measured by redox titration method. In detail, 20mL of 0.05mol/L iodine aqueous solution, 10mL of 10mg/mL dextran aldehyde solution and 20mL of 1 mol/L sodium hydroxide aqueous solution were charged into a 100mL Erlenmeyer flask, and stirred at 25°C for 15 minutes. Then, the solution was added with 15ml of 6v/v% sulfuric acid aqueous solution, and was titrated with 0.1 mol/L sodium thiosulfate aqueous solution. Time point at which reaction system becomes colorless and transparent was deemed as ending point, while starch aqueous solution was used as indicator.

As shown in Fig. 1, the amount of the introduced aldehyde groups increases linearly with increase of amount of the added sodium periodate. When the molar amount of the added sodium periodate per anhydro glucose unit is in a range of 0.05 to 1.0, the amount of the introduced aldehyde groups per anhydro glucose unit is in a range of 0.1 to 2. Thus, when one mole of sodium periodate is added with respect to one mole of anhydro glucose unit, two aldehyde groups are introduced to each anhydro glucose unit on average. Thus, it was revealed that efficient oxidation is achieved.

### 2. Measurement of cure time

Twenty grams of dextran (Wako Pure Chemical Industries, Ltd; Lot No. EWR5671) having weight-average molecular weight of 40,000 was dissolved in 100mL of distilled water. Then, 10g or 5g of sodium periodate was added and stirred at 50°C for 3 hours as to proceed reaction. After the reaction, obtained solution was subjected to dialysis for 24 hours by use of distilled water and dialysis membrane of fractioning molecular weight of 14,000, and thereafter being freeze dried. Thus, the dextran aldehyde was obtained. Each of dextran aldehydes was dissolved in distilled water to prepare 20wt% solution and was used as the first liquid of the two-liquid reactive adhesive.

Then, 25wt% polylysine aqueous solution (molecular weight of 4000; Chisso Corporation, Lot No.2050506, free amine) was added by 1 mL of acetic acid and 1.5mL of distilled water as to prepare 20wt% polylysine neutral aqueous solution. Meanwhile, polyethylene glycol amine having an amino group on each of two ends (two-functional PEG-NH₂, free amine) and having molecular weight of 3000 (the polyethylene glycol amine polymer not being part of the invention) was dissolved in distilled water as to prepare 30wt% and 50wt% aqueous solutions (free amine). Further, polyethylene glycol having an amino group on each end of four branches and having molecular weight of 5000 (four-functional PEG-NH₂, free amine) the polyethylene glycol amine polymer not being part of the invention) was dissolved in distilled water to prepare 50wt% aqueous solution. Each of these aqueous solutions of amino compounds was used as the second liquid of the two-liquid reactive adhesive.

Subsequently, 0.5mL of the second liquid of the two-liquid reactive adhesive was taken into glass test tube having diameter of 16mm, into which magnetic stirrer bar having a diameter of 4mm and a length of 10mm was putted, and was heated to 37°C and stirred at a rate of 100rpm. And, 0.5mL of the second liquid of the second liquid, which had been preheated to 37°C, was added to the solution by micropipette. Time length up to a timepoint at which the stirrer bar ceased its rotating as a result of curing was measured by use of a stopwatch. Table 1 shows results for the adhesive having the first liquid that is prepared by "10g addition" of sodium periodate; and table 2 shows that results for the adhesive having the first liquid that is prepared by "5g addition". In the tables 1 and 2, only those adopting the 20wt% neutral polylysine aqueous solution as the second liquid are Examples (Example 1 and Example 2 in the tables 1 and 2 respectively) of the invention. When solutions of the polyethylene glycol having amino end groups are used, strength of the gel adhesive layer was insufficient and the self-disintegration within time period of 1 day to 1-2 months was not achieved.

**Table 1. 10g NalO₄ addition as for the first liquid**

| (10g NalO₄ /20g Dextran 40K, 20wt% aq.soln.) | | | |
|---|---|---|---|
| Amino compound | Concentration (%) | Liquid property | Cure time (second) |
| Polylysine | 25 | Basic | <1 |
| 20wt% polylysine with 2% acetic acid | 20 | Neutral | 4.55±0.12 |
| Two-functional PEG-NH₂ | 50 | Basic | 10.51±0.19 |
| Two-functional PEG-NH₂ | 30 | Basic | 16.35±0.58 |
| Four-functional PEG-NH₂ | 50 | Basic | 6.30±0.21 |

**Table 2. 5g NalO₄ addition as for the first liquid**

| (5g NalO₄/20g Dextran 40K, 20wt% aq.soln.) | | | |
|---|---|---|---|
| Amino compound | Concentration (%) | Liquid property | Cure time (second) |
| Polylysine | 25 | Basic | 1.31±0.04 |
| 20wt% polylysine with 2% acetic acid | 20 | Neutral | 10.57±0.03 |
| Two-functional PEG-NH₂ | 50 | Basic | 23.38±2.59 |
| Two-functional PEG-NH₂ | 30 | Basic | 33.92±0.34 |
| Four-functional PEG-NH₂ | 50 | Basic | 18.51±0.31 |

As shown in Tables 1 and 2, gelling time of an adhesive formed of the four functional PEG-NH₂ as amino compound is shorter than that formed of two functional PEG-NH₂, which has smaller number of functionality. An adhesive formed of the polylysine, which has one amino group on every anhydro glucose unit on average, has the gelling time shorter than those on above. The larger the concentration of the amino compounds, the more rapid becomes the curing. The gelling time for adhesives of "10g NalO₄ addition" tends to become about half of that for adhesives of "5g NalO₄ addition". These results reveal that the curing time may be controlled by number of the aldehyde groups and categories and concentration of the amino compound.

### 3. Cytotoxicity test

Twenty grams of dextran (Wako Pure Chemical Industries, Ltd; Lot No.EWK3037) having weight-average molecular weight of 75,000 was reacted with 5g of sodium periodate, and the dextran aldehyde was obtained in accordance with methods in Section 2. Then, 20wt% aqueous solution was prepared as the first liquid.

Meanwhile, 10mL of 25wt% polylysine aqueous solution that was used in Section 2 was added with 0.5mL of succinic anhydride and 14.5mL of distilled water as to prepare 10wt% neutral polylysine aqueous solution, which was used as the second liquid (Reference Example 3). Meanwhile, about 0.5g of 30wt% formaldehyde aqueous solution and 0.5g of 25wt% glutaraldehyde aqueous solution were precisely weighed, and respectively used for preparing 25mL of aqueous solutions. By use of each of thus obtained aqueous solutions, cytotoxicity test was conducted in accordance with follows.

The cytotoxicity test was made according to methods described in J. Biomed. Mater. Res. 29,829-835(1995) and by use of a mouse established cell line L929. In detail, cell dispersion was prepared to be 10,000cells/mL; and 0.1mL of the cell dispersion was sowed in each well of the 96-well culture plate and incubated at 37°C for 3 days. Subsequently, each of the aqueous solutions mentioned above was diluted to one of various concentrations, and was charged into respective well by 0.1 mL. Then, incubation was further continued for 2 days. Subsequently, 0.1 mL of neural red culture-medium solution as prepared as 150 microgram/mL was added into the each well; and the incubation was made at 37°C for 3hours. After the incubation, culture medium was removed; and the cells were fixed by 1 wt% glutaraldehyde aqueous solution and thereafter air-dried. Subsequently, 0.1mL of water/ethanol solution (equal-volume mixture) having 1wt% of acetic acid was added; and then neural red molecules that had been taken into the cells were extracted. Light absorbency at 541 nm was measured and NR50 values were obtained from result of this measurement. The NR50 value indicates a concentration at which 50% of the cells are killed. The smaller the NR50 value becomes, the larger becomes the toxicity.

Table 3 shows the NR50 values of various compounds.

**Table 3. Cytotoxicity Evaluation Results**

| Sample | Component | NR50 value (µg/mL) |
|---|---|---|
| First liquid | Dextran aldehyde | 6,019±91 |
| Second liquid | Polylysine | >10,000 |
| | Formaldehyde | 1.7±02 |
| | Glutaraldehyde | 3.9±0.7 |

As shown in the table, the NR50 value of the dextran aldehyde was larger than 6000µg/mL and was less than 1/3500 of that of the formaldehyde and less than 1/1500 of that of glutaraldehyde. It was revealed that toxicity of the dextran aldehyde is trifle, even though the dextran aldehyde is also an aldehyde compound. Meanwhile, toxicity of the polylysine was revealed to be extremely low; and it is considered that each component of the adhesive has extremely high safety.

### 4. Evaluation of flexibility of the cured resin by use of a rubber glove

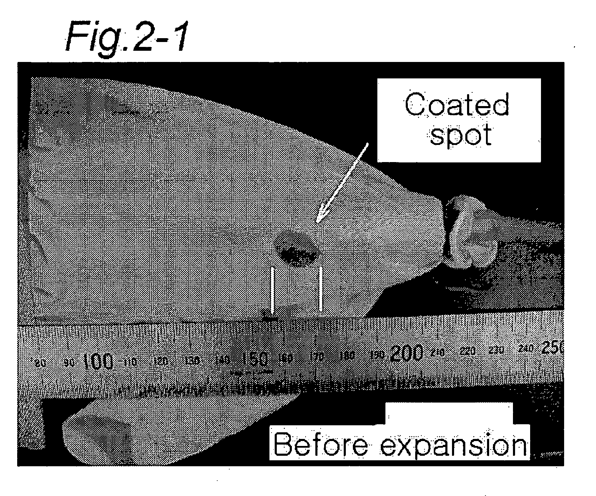

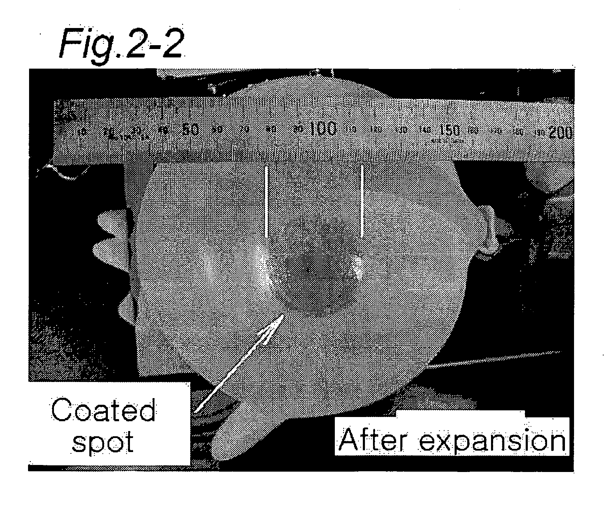

As the first and second liquids, adopted respectively were the 20wt% aqueous solution of the dextran aldehyde (20g of dextran is reacted with 10g of sodium periodate) and 20wt% neutral polylysine aqueous solution, which are obtained in Section 2. In other words, two-liquid adhesives of the Examples 1 and 2 were used. The first and second liquids, as are, were mixed together by a mixing device dedicated solely for such adhesives and then applied on a rubber glove for laboratory, by 0.5mL (0.25+0.25mL), as to be thinly spreaded. The adhesive layer was left standstill for about one minute as to achieve curing of the adhesive. Air is pumped into the rubber glove by use of air pump as to make expanding of the cured adhesive layer. Fig. 2 shows states of the cured adhesive before the expanding (a) and after the expanding (b). In the figure, what is stained with Blue No. 1 (Wako Pure Chemical Industries, Lot No.KLN3789) is a gel of the cured adhesive.

As shown in Fig. 2, the cured adhesive was enlarged by three times in diameter by pumping of the air, and then neither crack nor fracture was observed. This result reveals that the adhesive of this example (Example 1) is extremely pliable even after the curing. A gel resin layer having similar flexibility and toughness was also obtained; when molecular weight of dextran is varied to 40000, when addition amount of the sodium periodate per 20g of dextran is varied to 3g or 5g or the like, and when dextrin is used; though photos and data are omitted here.

### 5. Evaluation of adhesion strength by use of rabbit leather

The 20wt% dextran aldehyde aqueous solution obtained at Section 4 was used as the first liquid. Adopted as the second liquid were; the 20wt% neutral polylysine aqueous solution, 30wt% and 50wt% two-functional PEG-NH₂ aqueous solutions, and 50wt% four-functional PEG-NH₂ aqueous solution. Only the adhesive adopting the 20wt% neutral polylysine aqueous solution as the second liquid is of an example (Example 1) of the invention.

Defatted leathers (1X6 cm) from back skin of domestic rabbit were prepared, on surfaces of which about 10µL of the first liquid and about 10µL of the second liquid are successively applied on an area of 1X1 cm. After sufficient mixing, the defatted skin, which had same size and not been applied with the adhesive, was overlaid as to stick onto the one having been applied with the adhesive. Then, such stuck pair of the leathers was applied with 200g of load and was left as it is for 5 minutes. Then, the stuck pair of the leathers was immediately subjected to a peel test by a tensile testing machine ("Autograph AGS-5KNG", SHIMADZU RIKA CORPORATION) so that shear strength was applied by pulling at a rate of 10mm/min until the stuck pairs of skins were peeled apart. Load at a time of the peeling apart was taken as adhesion strength. For comparison, fibrin glue ("Bolheal", KAKETSUKEN in Japan) was tested in same manner with above. Table 4 shows these results.

**Table 4. Adhesion strength evaluation by use of rabbit leather**

| Sample | | Adhesion strength (g/cm²) |
|---|---|---|
| First liquid | Second liquid | |
| Dextran Aldehyde (5g NalO₄ /20g Dextran 40K, 20wt%) | Polylysine | 144.8±7.6 |
| | Two-functional PEG-NH₂, 30wt% | 10.6±0.8 |
| | Two-functional PEG-NH₂, 50wt% | 14.9±0.7 |
| | Four-functional PEG-NH₂, 50wt% | 160.5±0.9 |
| Fibrin glue | | 28.7±1.3 |

As shown in Table 4, the adhesives respectively adopting the polylysine aqueous solution and the four-functional PEG-NH₂ aqueous solution have adhesion strengths far higher than that of the conventional adhesive of fibrin glue.

### 6. Disintegration controlling of the cured resin by chitosan addition

The 20wt% dextran aldehyde aqueous solution obtained at Section 4 was used as the first liquid. Meanwhile, chitosan product of YAEGAKI Bio-industry, Inc., which has molecular weight of 100,000 and deacetylation degree of 80%, was dissolved in 5wt% acetic acid aqueous solution, as to prepare 5wt% chitosan solution. The 5wt% chitosan solution was mixed with the 20wt% neutral polylysine aqueous solution obtained in Section 4, in various volume ratios; and such mixtures were used as the second liquid. In other words, the adhesive of the Example 1 was modified such that the second liquid was partially replaced with the chitosan solution.

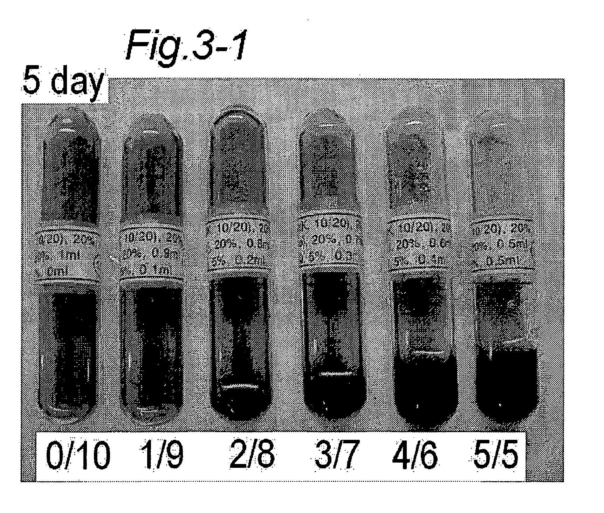

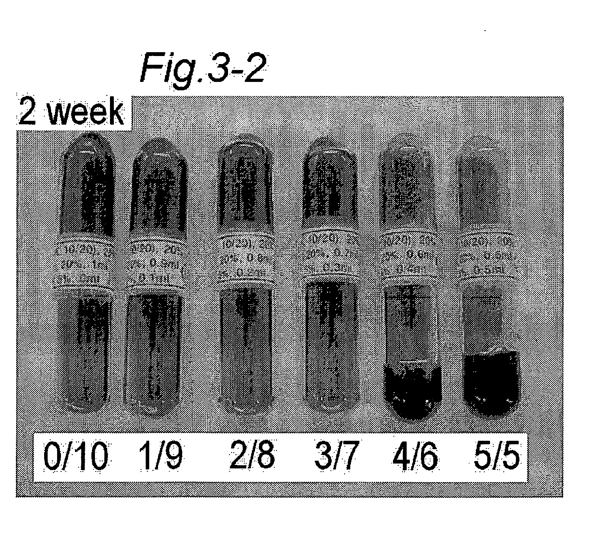

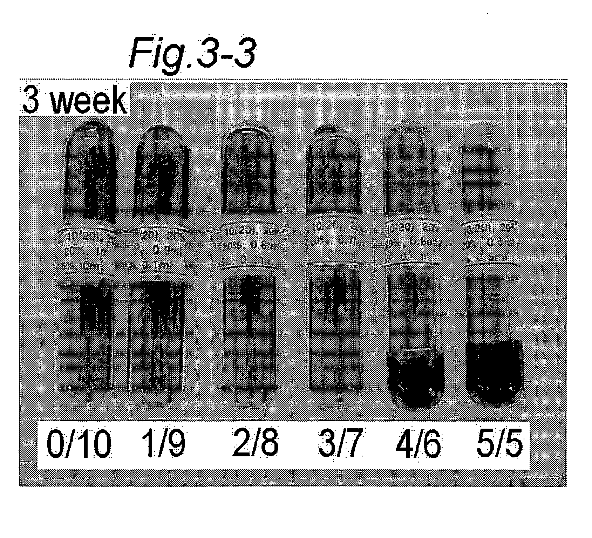

Subsequently, into a 16mm glass test tube, each of the first and second liquids was charged as it was, by 1 mL. After curing of the liquids, 3mL of phosphate buffer solution was added into each of the test tubes; and thereafter the test tubes were air-tightly sealed. Then, the test tubes were put into a dryer of 37°C, and disintegration of the adhesives was observed in a time course. Fig. 3 shows states of the adhesives after elapse of 5 days after the curing. On Fig. 3, denotation under each of the test tubes designates volume ratio between the 5wt% chitosan solution and the 20wt% neutral polylysine aqueous solution.

As shown in Fig. 3, the adhesives of the embodiments having small chitosan contents (volume ratio 0/10 and 1/9) completely disintegrated within 5 days. Increase of the chitosan content resulted in increase of remaining amount of the cured resin; and the adhesive of 5/5 volume ratio did not show disintegration even after elapse of 3 weeks. These results reveal that time-wise ratio of the disintegration or time period up to the fluidization of hydrogel resin is able to be controlled by specie of the amino compounds and mixture ratios.

As seen from these results, when the solution of chitosan having relatively small value of 100,000 was solely used as the second liquid of the two-liquid adhesive, the self-disintegration property of the invention was never observed. This fact indicates that no advantageous effect of the invention would be obtained, even when linear polymer of amino-group-containing units is used, unless; the polymer has molecular weight of no more than dozen thousands, especially no more than 20,000.

### 7. In vivo disintegration rate evaluation of the cured adhesive

As the first liquid, the 20wt% aqueous solution of dextran aldehyde having molecular weight of 75,000 used in Section 3 was adopted. The 10wt% neutral polylysine aqueous solution same as in Section 3 was prepared and used as the second liquid. Consequently, the adhesive of the Example 1 was adopted. For evaluating time-wise rate of in vivo disintegration of the cured adhesive, following test was made by use of female domestic rabbit having weight of 2.5-3.0kg.

Nembutal was injected through an ear vein by amount of 0.6mL per kilogram of body weight, and then Selactar was injected through leg muscle by amount of 0.1 mL per kilogram of body weight, as to achieve anesthesia. After opening of abdominal cavity, a filter paper (TOYO No.2) having a cut-out opening at a diameter of 10mm was pasted on a liver surface. Then, the first and second liquids were mixed with each other by use of a mixer device dedicated to such mixing; and 1mL of mixed liquid was applied on the liver surface. After the curing, the abdominal cavity was closed, and a time course of the disintegration of the cured resin was investigated.

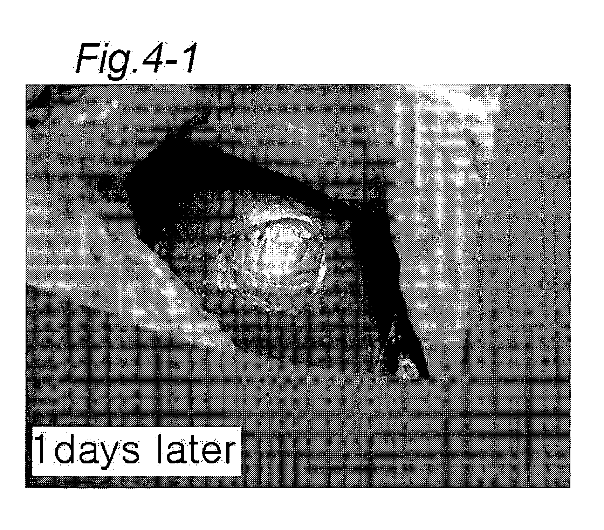

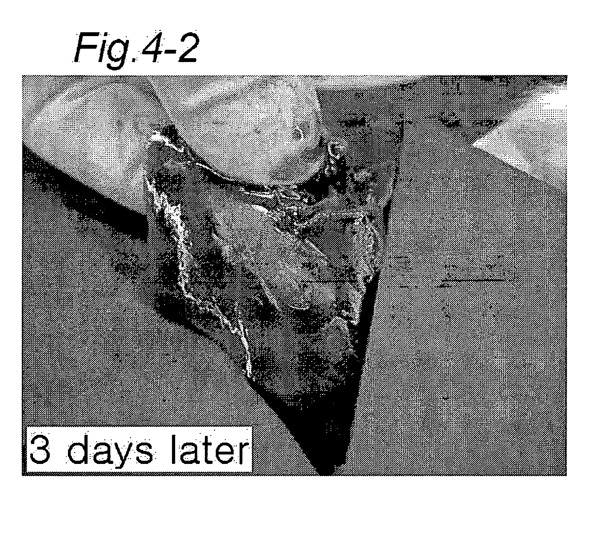

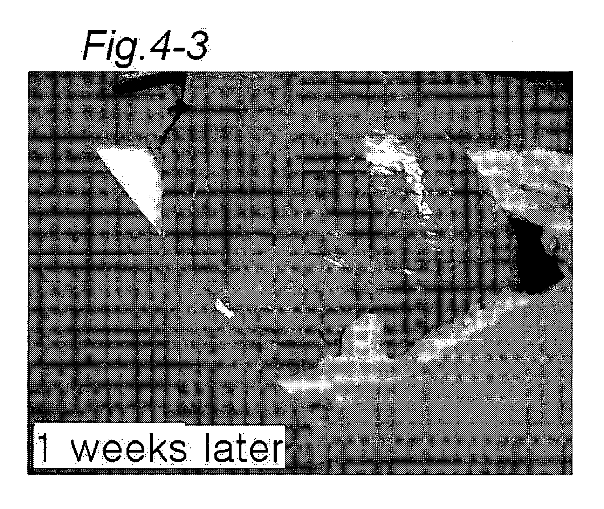

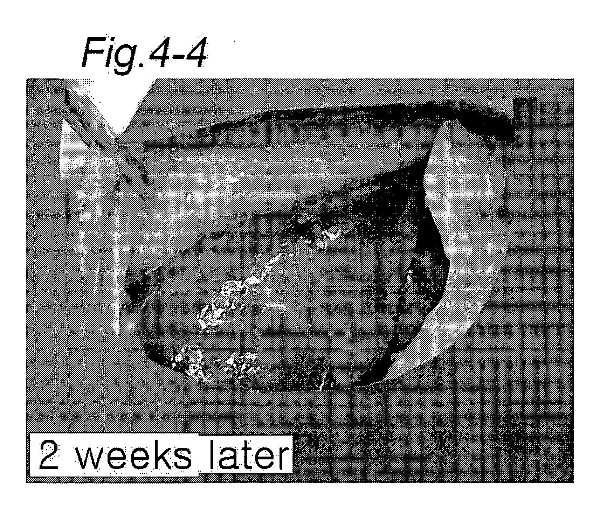

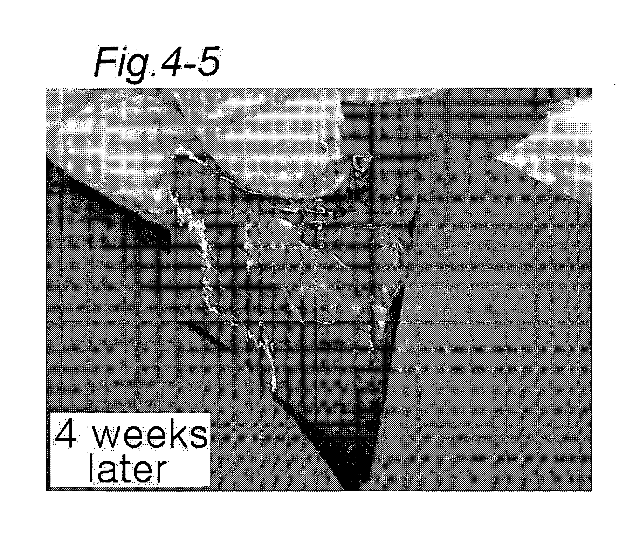

Figs. 4 show disintegration of the cured adhesive. It was determined that the disintegration gradually starts at elapse of 3 days after implanting of the adhesive and that about 90% of the cured adhesive had been disintegrated at an elapse of 4 weeks. Neither tissue reaction nor adverse effect that would be feared especially from image of tissue section was observed, as to show high safety of the adhesive. On course of the disintegration test, there was observed neither decrease of appetite of the rabbit nor other adverse effect due to implanting of the adhesive.

### 8. Controlling of disintegration of the cured adhesive by addition of citric acid

The 20wt% dextran aldehyde aqueous solution obtained in Section 4 was used as the first liquid. Meanwhile, 10mL of the 25wt% polylysine aqueous solution having basicity used in Section 2 was added with acetic acid and citric acid, by combined amount of 1 mL, with various ratios between the acids; and further added with 0.5mL of distilled water as to form a 20wt% neutral polylysine aqueous solution, which was used as the second liquid. In other words, the two-liquid adhesive of the Example 1 and the two-liquid adhesive of the Reference Example 3 (Section 3) were mixed in ratios of 8/2, 6/4, 4/6 and 2/8, as to prepare adhesives of embodiments of the invention. The adhesive same as the Example 1 solely using acetic acid (10/0) for neutralization, as well as the adhesive same as the Reference Example 3 solely using citric acid (0/10), are prepared and used for comparison.

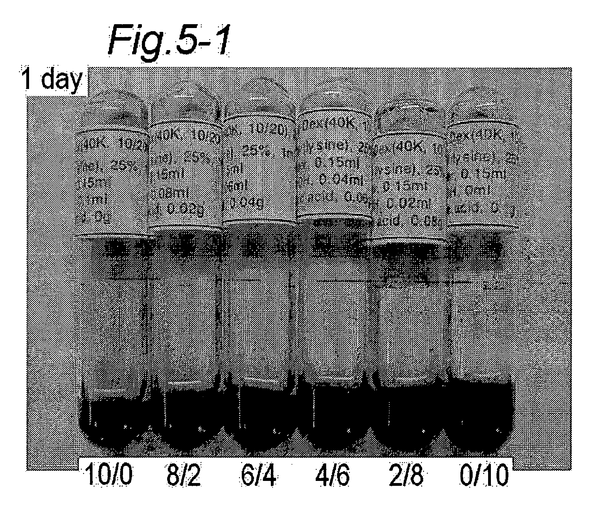

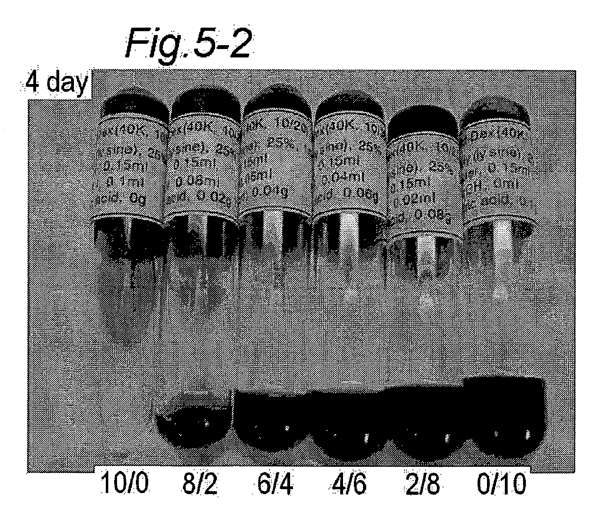

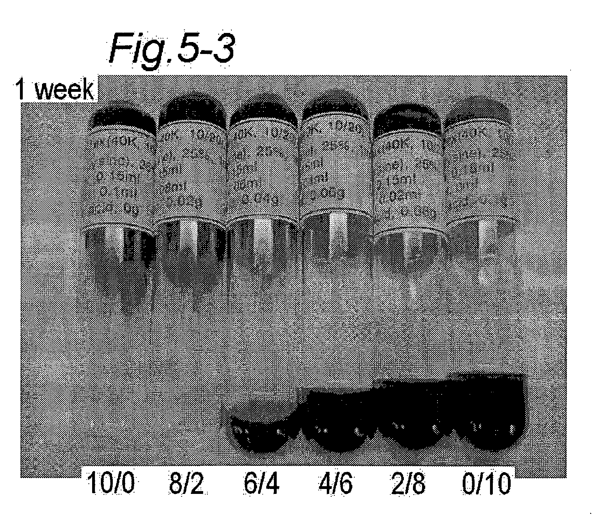

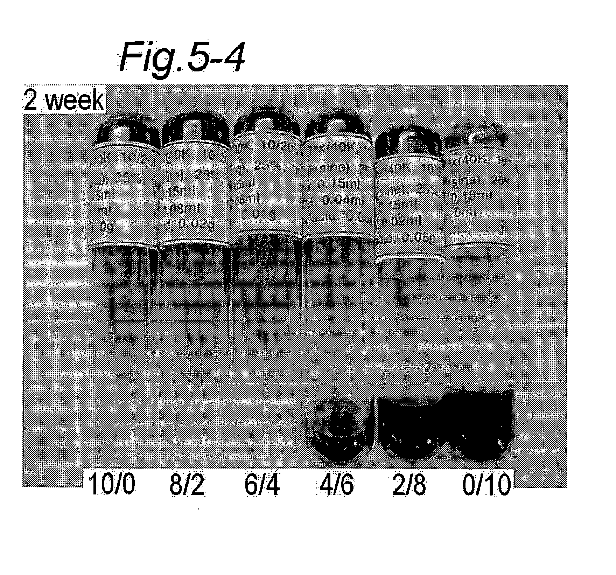

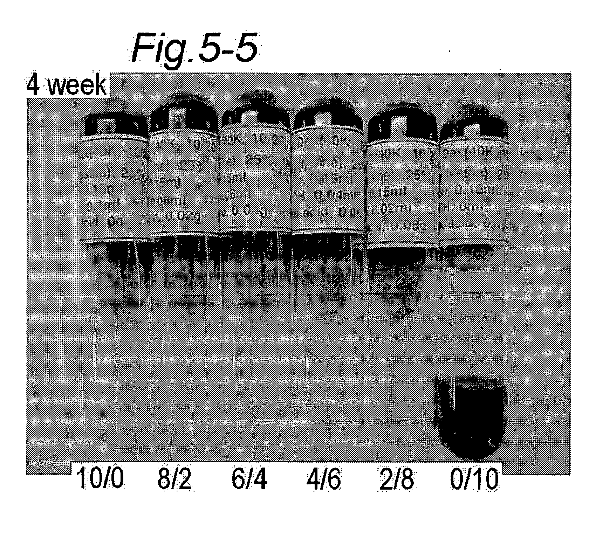

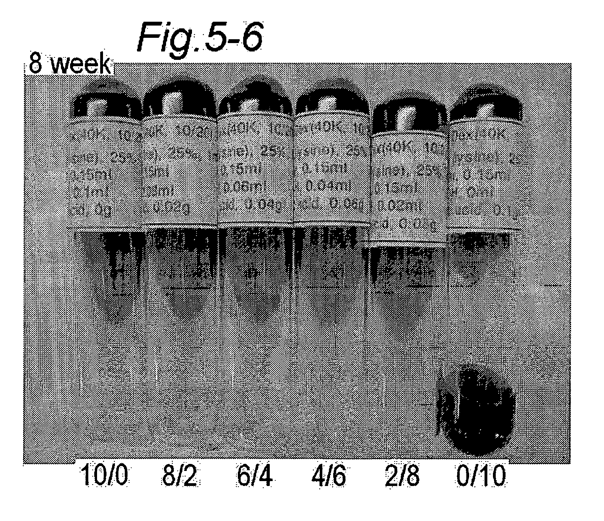

Subsequently, disintegration rate of the cured adhesive was evaluated by a method described in Section 6. Results of the evaluation are shown in Fig. 5. Denotations in the figure indicates weight ratios between acetic acid and citric acid; and as for left-end one, pH adjustment is made solely by acetic acid, while solely by citric acid as for right-end one. When acetic acid was solely used for pH adjustment of the polylysine aqueous solution, the cured adhesive completely disintegrated at elapse of 4 days. The disintegration was delayed by increase of citric acid. When citric acid was solely used for pH adjustment of the polylysine aqueous solution, the cured adhesive kept its original shape even at elapse of 2 weeks and required no less than 2 months for complete disintegration. These results indicate that disintegration rate is easily controlled by selecting species and amount of pH adjuster.

### 9. Conversion of polylysine to high-molecular-weight one by heating and its effect on disintegration rate of the cured resin.

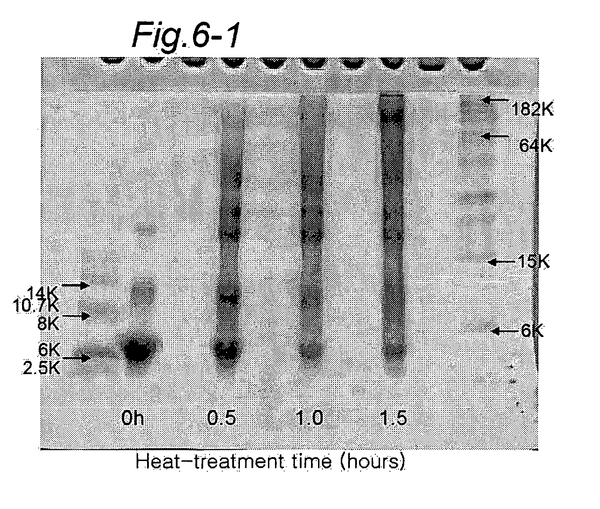

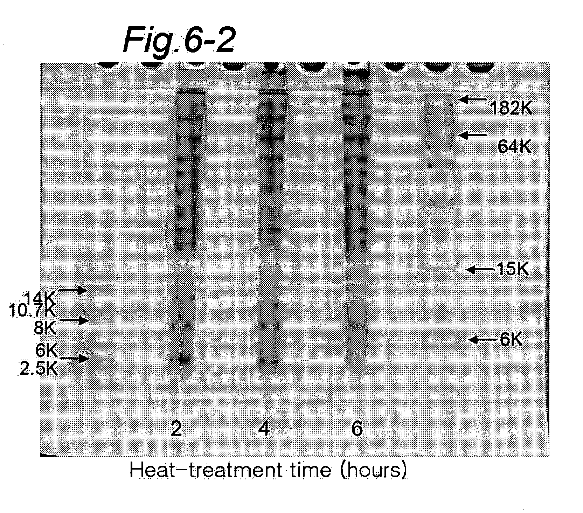

Powder of epsilon-poly-L-lysine having molecular weight of 4000 (Chisso Corporation, Lot No.20211023F) was subjected to vacuum drying (heat treatment) at 180°C. Molecular weight of the heat-treated ε-poly-L-lysine was evaluated by gel electrophoresis, in which adopted for the gel electrophoresis were 15% SDS polyacrylamide gel as electrophoresis gel as well as "Running buffer solution" (Nacalai Tesque, Inc.; 0.25moL/L-Tris, 1.92moL/L-glysine 10g/L-SDS) as mobile phase. Gel electrophoresis apparatus of NIHON EIDO CO., LTD was used and gel electrophoresis is made under electric current of 50mA. Adopted as markers were; "Peptide molecular weight marker" (Daiichi Pure chemicals Co., Ltd.; Mw=2,512 to 16,950; Lot No.024RJZ) and "Protein Ladder" (Invitrogen Corporation; Mw=6,000 to 181,800; Lot No.1283301A); and adopted as stain was "Coomassie brilliant blue" (Invitrogen Corporation; R-250). As a result, electrophoresis pattern as shown in Fig. 6 was obtained. For non-treated sample (time of the treatment is zero), main component appears between 2.5k and 6K, as to accord with described value of the molecular weight, that is 4000, on a catalogue of Chisso Corporation. The other components were discontinuous, presumably due to association of the component having molecular weight of 4000. Heat treatment led to disappearance of spots and remarkable increase in molecular weight. Heat treatment exceeding 1.5 hours led to appearance of components exceeding the maximum molecular weight marker of 182K; and increase of heat treatment led to increase of high-molecular-weight components.

Heat-treated powders of the polylysine obtained in above were dissolved in distilled water as to prepare 10wt% solutions while including 4wt% of acetic acid, and thereby prepare the second liquids.

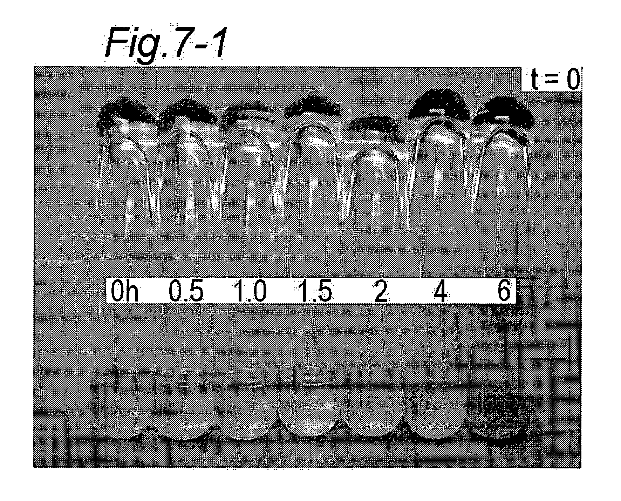

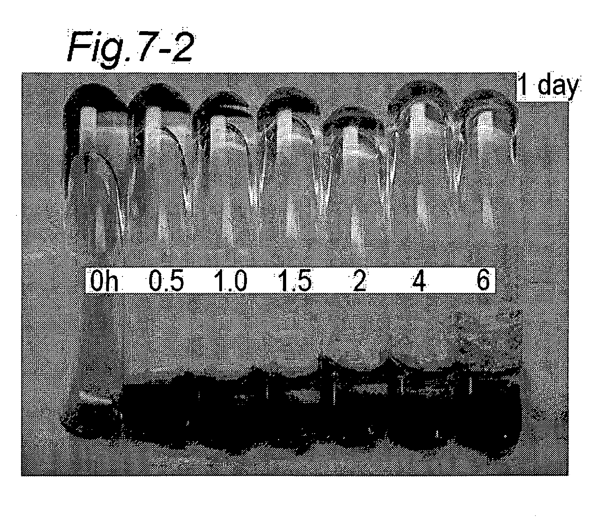

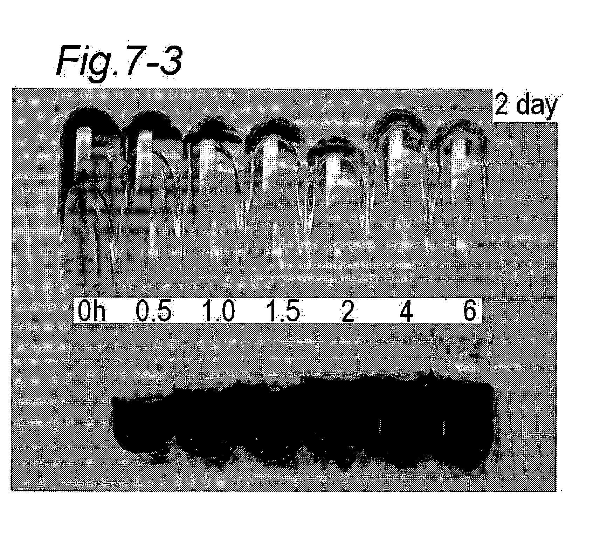

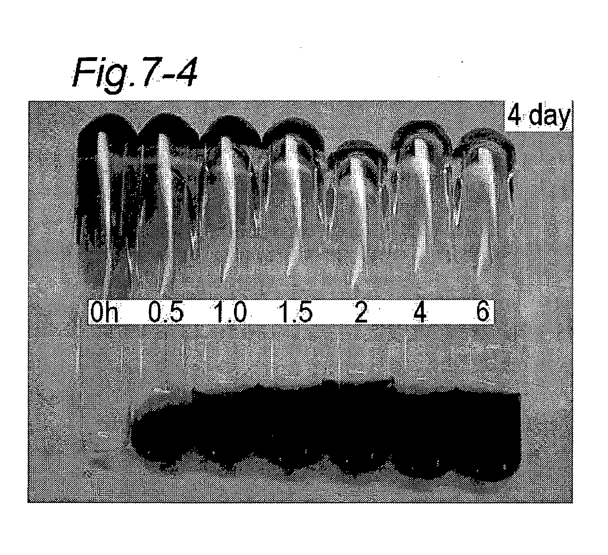

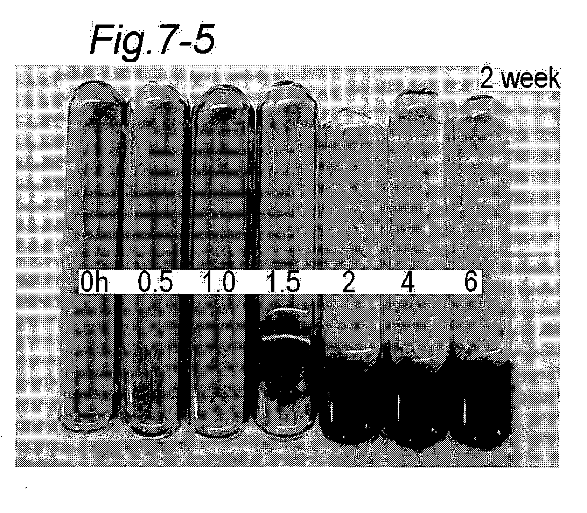

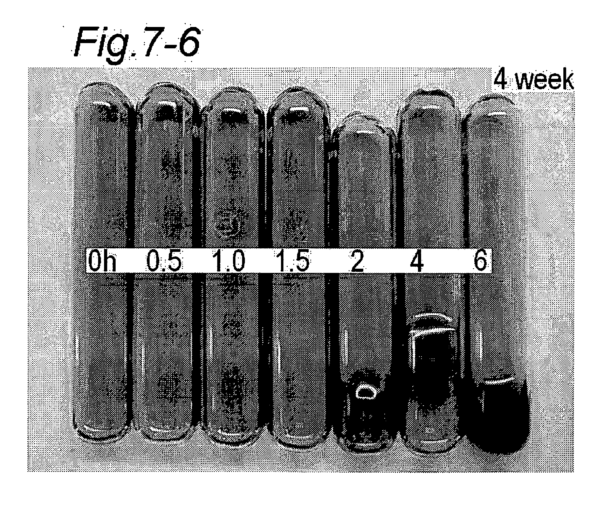

As the first liquid, the 20wt% aqueous solution of dextran aldehyde having molecular weight of 75,000, which was used in Section 3, was adopted. After mixing the first and second liquids, disintegration rate of the cured adhesive was evaluated by the method described in Section 6. Results of the evaluation are shown in Fig. 7. Time period required for the disintegration increases with increase of the heat-treating time period and thereby with increase of molecular weight. When the heat-treating time period is zero, the cured adhesive was disintegrated by two days. When the heat-treating time period is 1.5 hours, two weeks was required for the disintegration; while 6hours of the heat treating made require no less than one month for the disintegration.

As mentioned in Section 8, disintegration time period of the cured resin may be easily delayed by selection of pH adjuster (among acetic acid, citric acid and so on). Hence, it is considered that adopting of ε-poly-L-lysine without heat treating enables controlling of the disintegration time period freely in a range from short time up to a long time. On contrary, when adopting of ε-poly-L-lysine having been subjected to long heat treatment, it is considered that such controlling becomes difficult. However, heat treatment of around 0.5 hour or less, at 180°C, for example would be adoptable as a way for controlling the disintegration time period.

Taking account of results in Section 6, in which the chitosan having molecular weight of 100,000, would lead following; molecular weight of the amino-group-containing polymer have to be no more than dozen thousands in order to achieve sufficient self-disintegration of the invention.

### 10. Applying of electron radiation sterilization

As the first liquid, the 20wt% aqueous solution of dextran aldehyde having molecular weight of 75,000 used in Section 3 was adopted. The 10wt% neutral polylysine aqueous solution same as in Section 3 was prepared and used as the second liquid. The first and second liquids are filtered through a syringe filter (Dismic 25AS020AS; ADVANTEC MFS, INC or Toyo Roshi Kaisha, Ltd.) having apertures of 0.2µm diameter then be respectively charged into 5mL glass ampoules, which are then sealed. For each pair of the first and second liquids, sterilization was made at 25°C under condition shown in left-hand end of Table 5. After sterilization, curing time was measured by the method shown in Section 2 as to reveal effect of the sterilization. These results are shown in Table 5.

**Table 5. Varying of curing time due to radiation irradiation**

| Sterilization | Curing time/second* | Difference/second |
|---|---|---|
| No sterilization | 13.04±0.20 | 0 |
| Gamma radiation (25KGy) | 13.65±0.06 | 0.62 |
| Electron radiation (20KGy) | 10.66±0.26 | -2.38 |
| Electron radiation (40KGy) | 8.25±0.07 | -4.79 |

| | | |
|---|---|---|
| * data=average±S.D. (n=3) | | |

As shown in the table, the gamma radiation induced a slight delay of the curing while, on contrary, the electron radiation induced shortening of the curing time. Thus, it is presumed that the electron radiation induces neither decrease of molecular weight nor alteration in respect of each component of the adhesive and has wide variety of applicability as a way for sterilization of the adhesives of the invention.

### 11. Softness evaluation of the cured adhesive by compression test

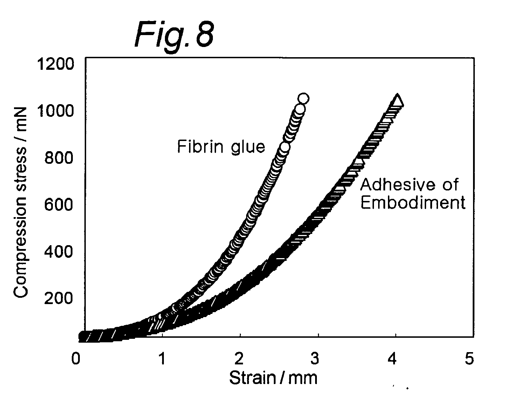

As the first liquid, the 20wt% aqueous solution of dextran aldehyde having molecular weight of 75,000 used in Section 3 was adopted. The 10wt% neutral polylysine aqueous solution same as in Section 3 was prepared and used as the second liquid. The first and second liquids were mixed with each other by the mixing apparatus dedicated to such mixing; and 2mL of thus obtained mixture was poured into each well having 2cm² of bottom area, on a 24-well plate for cell culture. After leaving at 25°C for 2 minutes, a hemisphere having diameter of 6.6mm was pressed onto the cured adhesive at a rate of 10mm/min by use of a tensile test machine (ADS-5D of SHIMADZU CORPORATION), as to measure compression stress and thereby to evaluate pliability of the cured resin. As a comparative adhesive, the fibrin glue ("Bolheal", KAKETSUKEN in Japan) was subjected to an evaluation procedure same as above. Fig. 8 shows stress-strain curve of the results.

In the figure, each plot represents a penetration distance (mm), by which the hemisphere was penetrated into the cured resin, and a corresponding stress value (mN). Inclination of a plotted curve for the adhesive of the embodiment is as much as about half of that for the fibrin glue, in a region at afterward of about 1 mm of the penetration distance, while initial inclinations of the two curves make only a small difference. This result indicates that the cured adhesive of the invention is softer than cured fibrin glue.

Though data is omitted here, the strain-stress curve of the adhesive of the invention at the compression test was varied by modifying the addition amounts of sodium periodate into 3g or 10g, with respect to 20g of dextran. Hence, by modifying of extent of the aldehyde groups introduction, cross-linkage density at a time of the curing is freely changed as to freely control softness of the cured gel resin layer.

### 12. Evaluation of adhesion strength by use of cattle leather

As the first liquid, the 20wt% aqueous solution of dextran aldehyde having molecular weight of 75,000 used in Section 3 was adopted. The 10wt% neutral polylysine aqueous solution same as in Section 3 was prepared and used as the second liquid. In other words, the adhesive of Reference Example 3 was adopted. Adhesion test was made by using cattle leather as adherents for evaluating adhesion strength of the adhesive. Cattle leather (TRUSCO Corporation; JT-5L, long-sleeve leather glove for worker) is sliced to strips of 1cm×5cm; and a pair of leather strips was bonded by an adhesion area of 1cm×1cm and was then left to stand still under 100g of load as to achieve the curing. Thus obtained bonded pair of strips was subjected to shear adhesion strength test by use of the tensile test machine used in Section 11 and by causing shear motion at a rate of 10mm/min; and adhesion strength at a time of peeling apart was obtained (n=5). As a comparative adhesive, the fibrin glue ("Bolheal", KAKETSUKEN or The Chemo-sero-therapeutic Research Institute, in Japan) was subjected to an evaluation procedure same as above.

Resultantly, adhesion strength was 2024±563g/cm² for the adhesive of the embodiment (Reference Example 3) and was 519±136g/cm² for the fibrin glue. As for the adhesive of the embodiment, its average adhesion strength was about 4 times of that of the fibrin glue and most of samples exhibited adhesion strength no less than 1 kg/cm². On contrary, adhesion strength of the fibrin glue was generally low and varied widely, due to too short curing time and thereby to difficulty in preparing the bonded samples. Curing time measured by a method of Section 2 was about 10 seconds for the adhesive of the embodiment and was no more than a second for the fibrin glue.

### 13. An example of use on digestive surgery Haemostasis on liver

As the first liquid, the 20wt% aqueous solution of dextran aldehyde used in Section 4 was adopted. Adopted respectively as the second liquids were; the 20wt% neutral polylysine aqueous solution and the 50wt% two-functional PEG-NH₂ (free amine) aqueous solution, both of which had been obtained at Section 2. The adhesive adopting the neutral polylysine aqueous solution as the second liquid is equivalent to the adhesive of the Example 1 in Section 2.

Abdominal cavity of a domestic rabbit that had been sacrificed was opened along a median line as to expose a liver. By use of a surgical scalpel, a wound (incision) of about 2cm length and about 5mm depth was made as to cause continuous bleeding from the incision. Subsequently, the first and second liquids were mixed by equal volumes, by use of a mixing device dedicated to such mixing; and the mixture was applied on the incision.

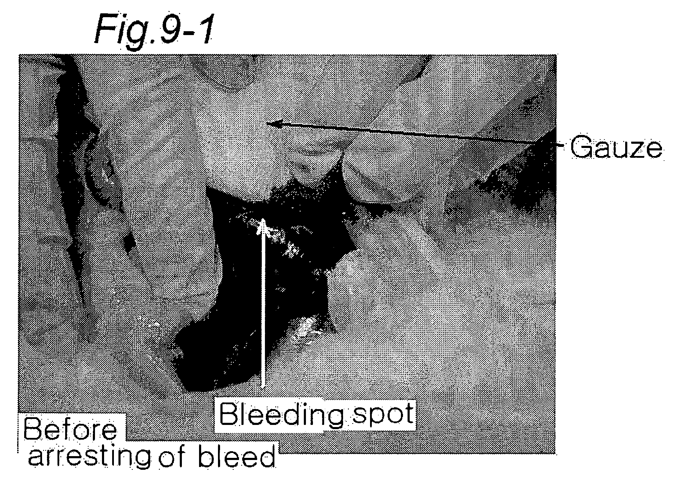

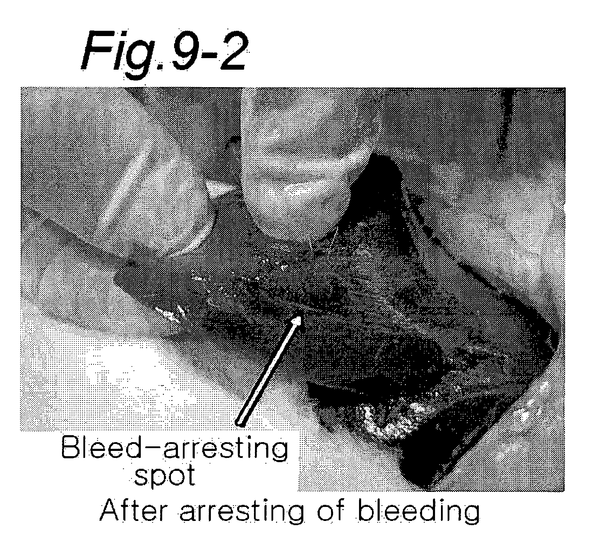

Resultantly, a reliable arrest of bleeding was observed; within 10 seconds by the adhesive (Example 1) adopting the neutral polylysine aqueous solution as the second liquid; and within 30 seconds by the adhesive adopting the two-functional PEG-NH₂ aqueous solution as the second liquid. Fig. 9 shows the liver before and after the haemostasis by use of the adhesive adopting the polylysine solution as to indicate achieving of effective haemostasis. Moreover, a film of the adhesive was firmly fixed on the liver. Hence, it was revealed that the adhesive of this example exhibits excellent effect as haemostasis agent in digestive surgery.

### 14. An example of use on cardiovascular surgery Prevention of tissue adhesion

As the first liquid, the 20wt% aqueous solution of dextran aldehyde having molecular weight of 75,000 used in Section 3 was adopted. The 10wt% neutral polylysine aqueous solution same as in Section 3 was prepared and used as the second liquid. In other words, the adhesive of Reference Example 3 was prepared.

Meanwhile, 20g of dextrin having molecular weight of 7000 (Wako Pure Chemical Industries, Ltd; Lot No.EWQ7180) was reacted with 5g of sodium periodate; and dextrin aldehyde (aldehyde-groups-containing dextrin) was obtained by the method of Section 2. Subsequently, 20wt% aqueous solution of the dextrin aldehyde was prepared and used as the first liquid of the adhesive. And, 10mL of 25wt% polylysine aqueous solution that was used in Section 2 was added with 0.5mL of succinic anhydride and 14.5mL of distilled water as to prepare 10wt% neutral polylysine aqueous solution, which was used as the second liquid. Thus, a dextrin-based example of the two-part adhesive was obtained.

In order to confirm adhesion-prevention effect in cardiovascular surgery, pericardium of a rat was cut as opened. Then, surface of left ventricle was rubbed 100 times with a gauze pad as to make a condition for inducing the tissue adhesion. The rubbed surface was coated with each of the adhesives of various compositions; and then thorax was closed up. Four weeks later, the thorax was opened up and extent of tissue adhesion was evaluated (n=5) by 5 scales as to be ranked at either of 0, 1, 2, 3 and 4, in which larger number indicates higher extent of tissue adhesion. As an adhesive of a comparative example, fibrin glue ("Bolheal", KAKETSUKEN in Japan) that has been in clinical use as tissue-adhesion inhibitor was adopted. Obtained scales are as follows: 2.4±0.5 for non-treatment; 1.2±0.4 for fibrin glue; 3.2±0.4 for the adhesive of the Reference Example 3 (Section 3); and 1.2±0.4 for the dextrin-based adhesive of the embodiment.

Disintegration rate evaluation according to the method of Section 6 revealed the disintegration period of; one month for the adhesive of the Reference Example 3 (Section 3); and 2 to 3 days for the dextrin-based example of the adhesive. These results revealed that disintegration of the dextrin-based example of the adhesive is extremely rapid. In view of these and foregoing results, tissue adhesion would be severer when the adhesive having a long disintegration period was used than when no adhesive is applied; and extent of the tissue adhesion at an occasion the gel of the adhesive (Reference Example 3) having a short disintegration period was used was almost same with that at an occasion fibrin glue was used. Thus, it was revealed that the adhesive having a short disintegration period is highly effective for curbing tissue adhesion on the heart.

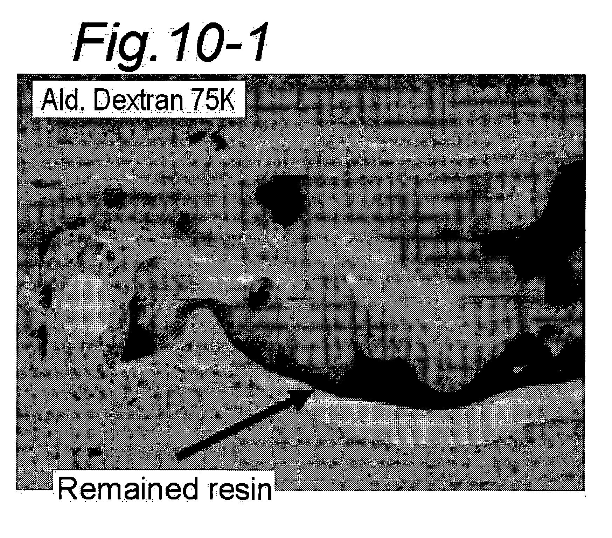

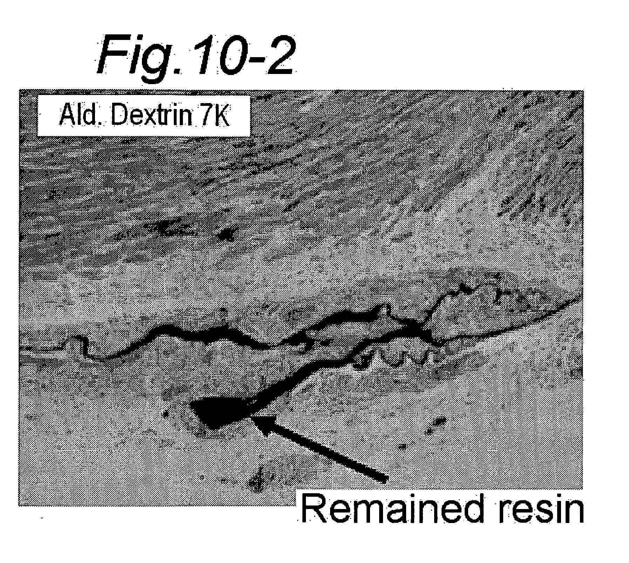

Fig. 10 shows an image of a tissue section that has been stained with Masson-Trichrome and taken from an affected part 4 weeks after surgery. As indicated by an arrow in the figure, remaining of the cured resin is seen on the image. At a time of surgery, epicardium on surface of the heart was rubbed by a gauze pad as to make a wound and thereby a tissue-adhesion-induced area. By such rubbing, the epicardium there was presumably ripped out near completely, as to expose cardiac muscles; and coating by the adhesive was made on such exposed part. A tissue enclosing the adhesive was formed after the coating. While amount of the coating was same between the adhesives, remaining amount of cured resin was very small for the dextrin-based adhesive of the embodiment due to high disintegration rate, compared to an occasion the adhesive of the Reference Example 3 was used. Presumably due to this, tissue adhesion was slight when the dextrin-based one was used. Hence, it is revealed that the adhesives of the embodiments are effective as the tissue-adhesion inhibitor for cardiovascular surgery because disintegration rate is easily controlled.

### 15. Other example of use on cardiovascular surgery Haemostasis

The 20wt% dextran aldehyde aqueous solution that had been used in Section 8 was prepared by use of dextran having molecular weight of 40,000, and was used as the first liquid. The 20wt% neutral polylysine aqueous solution used in section 8 was prepared and used as the second liquid (4% citric acid content). In other words, a two-liquid adhesive equivalent to the Reference Example 3 was prepared. The adhesive of Reference Example 3 has almost same property and performance with the adhesive of the Examples except that; disintegration is somewhat slower in the Reference Example 3 than in the Examples.

Following experiment was made for confirming haemostasis effect in cardiovascular surgery.

Anesthesia is made by follows; a rat is left in an ether atmosphere for 30 seconds, then a tube is inserted into a bronchus and making the rat inhaling isoflurane. Free wall of left ventricle of the rat was sutured up as to halt blood flow, and a pinhole was opened by picking with a 19-gage needle. Suture was temporarily loosen as to confirm occurrence of pulsative bleeding and then stitching up was made again to completely interrupt blood flow. Blood around the pinhole was removed, and a paper having a hole slightly larger than the 19 gage was put on the heart as to prevent flowing out of blood and the adhesive to surrounding.

Onto the pinhole, 0.4mL of the adhesive was applied by use of a syringe that is for injecting insulin. Three minutes later, a gauze pad was put on as to absorb oozed-out blood. Then, the suture was loosen and kept loosen for three minutes. Here, weight of the the gauze pad had been measured on before hand, and weight increase after absorption of blood was taken as amount of bleeding. For comparison, fibrin glue ("Beriplast", KAKETSUKEN in Japan) was tested in same manner with above. Numbers of rats used for this experiment were 6 for non-treatment, 5 for the adhesive of the embodiment and 5 for the fibrin glue.

Amounts of bleeding were 1.1±0.5g for non-treatment, 0.4±0.1g for the adhesive of the embodiment and 0.9±0.5g for the fibrin glue. The amount of bleeding for the adhesive of the embodiment was significantly smaller (p=0.02) than that for the fibrin glue. When the fibrin glue was used, two ways of bleeding were observed; by breaking out through the cured resin and by oozing through a fissure between periphery of the cured resin and a tissue. When the adhesive of the embodiment was used, bleeding only by oozing through the fissure was observed and amount of the oozing was small. Such difference of manner of bleeding is presumed to cause difference in amount of bleeding. Thus, the adhesive of the embodiment was revealed to be effective as haemostasis agent for cardiovascular surgery.

### 16. An example of use in respiratory surgery Obturation of air leakage from lung

As the first liquid, the 20wt% aqueous solution of dextran aldehyde having molecular weight of 75,000 used in Section 3 was adopted. The 10wt% neutral polylysine aqueous solution same as in Section 3 was prepared and used as the second liquid. In other words, the adhesive of Reference Example 3 was prepared.

To confirm obturation of the air leakage from lung, following experiment was made by use of a beagle dog. In accordance with common procedures, conducted were anesthesia, endotracheal intubation, and then opening of thorax; and a pleural deficient area of 3cmx3cm was cut out on right lung by use of an electrical scalpel. Physiological saline was sprayed on the deficient area, and pressure of respirator was increased, as to confirm occurrence of air leakage. Subsequently, by use of a mixing device dedicated to mixing of two-part liquids, about 2mL of the adhesive was dropped on the deficient area and rubbed to be speraded thereon with fingers for 10 seconds. Two minutes later, thorax was filled with physiological saline and a leakage test was made. As a comparative adhesive, the fibrin glue ("Bolheal", KAKETSUKEN in Japan) was used and fibrinogen solution was rubbed to be spreaded on the deficient area by fingers and then further applied thereon by use of a sprayer kit (rub & spray method). In regard of using of fibrin glue, leakage test was made 5 minutes after the application, in accordance with science literature(s).

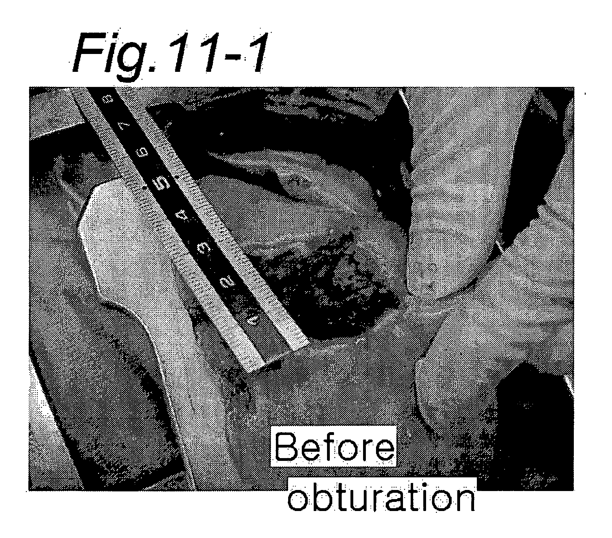

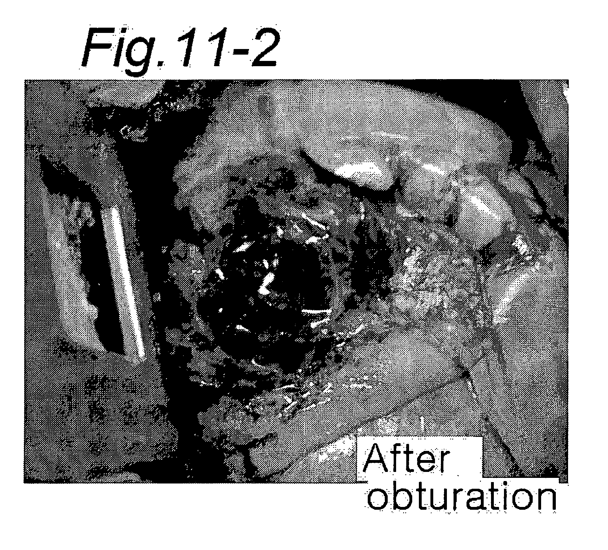

Pressure at which air leakage was observed was 35.4±6.8cmH₂O for the adhesive of the embodiment and was 33.3±4.8cmH₂O for the fibrin glue ("Bolheal"), and thus no big difference was recognized between them. Nevertheless, manners of air leakage greatly differed. As for fibrin glue, fibrin mass as a whole was peeled off from the lung as to expose the deficient area and thereby cause the air leakage (with large bubble). By the adhesive of the embodiment, the deficient area was surely kept. Pinholes are formed around the cured adhesive, and only very small bubbles were observed. Fig. 11 shows surface of the lung before and after coating of the adhesive of the embodiment and indicates that the deficient area is covered by the cured adhesive as a result of the coating of the adhesive. Progresses after the surgery were observed to be favorable both by the adhesive of the embodiment and by the fibrin glue. Theses results indicated that the adhesive of the embodiment is highly effective in respiratory surgery on purpose of air leakage obturation.

### 17. Reference Example 1 Polyethylene glycol having end amino groups

The 20wt% dextran aldehyde (MW 40,000) aqueous solution obtained at Section 4 was used as the first liquid. As the second liquid, adopted was the 50wt% aqueous solution of polyethylene glycol amine having an amino group on each of two ends (two-functional PEG-NH₂, free amine) and having molecular weight of 3000, which was obtained by dissolving in distilled water as Section 2.

Subsequently, time course of disintegration of the cured adhesive was observed by a method described in Section 6. Resultantly, the cured adhesive was completely fluidized three hours after the curing. The two-functional PEG-NH₂ has amino groups only on two ends even though having molecular weight of 3000, and hence, reaction takes place on the two ends. Thus, density of cross linkage would become very small; and this is presumed to be cause of rapid disintegration.

By results of this section and results of Section 2 on polyethylene glycol amine, it is supported that the amino-group-containing polymer has to be a polymer chain of amino-group-containing units.

### 18. Reference Example 2 Chitosan oligosaccharide

The 20wt% dextran aldehyde (MW 40,000) aqueous solution obtained at Section 4 was used as the first liquid. Meanwhile, powder of low-molecular-weight chitosan ("Y.H. Chitosan Oligosaccharide", YAEGAKI Bio-industry, Inc.; Lot. 000522; lactate, mixture of di- to hexa- saccharides, MW 700) was dissolved in distilled water as to prepare 50wt% aqueous solution, which was used as the second liquid.

Subsequently, the first and second liquids were mixed in a test tube for the reaction as in same manner with the Reference Example 1; and no curing was made even 7 hours later. Because of this, the test tube was further charged with 1 mL of the first liquid and with 100mg of the powder of "Y H. Chitosan Oligosaccharide" instead of the second liquid. Curing was observed 30miniutes later. Then, 3mL of phosphate buffer solution was added into the test tube, which was then sealed. And, time course of disintegration of the cured resin was observed. Resultantly, the cured resin was completely fluidized by 6 hours. Such rapid disintegration and fluidization are presumably due to follows. The "Y.H. Chitosan Oligosaccharide" has too low molecular weight, although having one amino group at each unit of sugar residue. Hence, gel would not be formed by use of 50wt% solution. Even by using of powder of the "Y.H. Chitosan Oligosaccharide", effective gel network structure would not be formed.

Results of using the chitosan oligosaccharide reveal that; molecular weight of a chain polymer of amino-group-containing units has to be at least 1000 when for achieving proper disintegration time period of one day or more. Results of using the chitosan oligosaccharide induce the assumption that; low-molecular-weight chitosan would make a behavior similar with that of the low-molecular-weight polylysine or the like. Hence, when taking account of results of Section 6 for chitosan addition, it is conceived that; the advantageous effect peculiar to the invention would be obtained only when molecular weight of chain polymer of the amino-group-containing units is in a range 1000 to 20,000.

### Brief descripion of the drawings

Fig. 1 is a graph showing a relationship between addition amount of sodium periodate and introduced amount of aldehyde groups (Section 1);
Figs. 2-1 and 2-2 are photographs showing an expansion of cured matter of adhesive that has been applied on a rubber glove (Section 4);
Figs. 3-1 to 3-3 are photographs showing a time course of disintegration of cured resins of adhesives that have been added with chitosan, up to three weeks after the curing (Section 6);
Figs. 4-1 to 4-5 are photographs showing a time course of disintegration of cured adhesive being on a liver of domestic rabbit (Section 7);
Figs. 5-1 to 5-6 are photographs showing a time course of disintegration of cured resins of adhesives, pH of each of which has been adjusted by a mixture of acetic and citric acids (Section 8);
Figs. 6-1 and 6-2 are electrophoresis patterns of polylysine that has been heat-treated (Section 9);
Figs. 7-1 to 7-6 are photographs showing a time course of disintegration of the cured resin of adhesive that has been prepared by use of heat-treated polylysine (Section 9);
Fig. 8 shows stress-strain curves for the cured resins of the adhesives (Section 11);
Figs. 9-1 to 9-2 are photgraphs showing a manner of haemostasis on a rabbit liver by adhesive of the embodiment (Section 13);
Figs. 10-1 to 10-2 are tissue's sectional images of an affected part 4 weeks after applying the adhesive on a tissue-adhesion-induced area (Section 14);
Figs. 11-1 to 11-2 are photographs showing a manner of obturation of air leakage from an artificially-induced deficient area, by use of the adhesive of the embodiment (Section 16).

## Claims

1. Medical-use two-component adhesive comprising: a first liquid comprised of aqueous solution of aldehyde-groups-introduced alpha-glucan having a weight-average molecular weight in a range of 1000 to 200,000 and a second liquid comprised of aqueous solution of amino-groups-containing polymer that is at least one selected from a group consisting of epsilon-poly-L-lysine, alpha-poly-L-lysine, chitosan oligomer and degraded chitosan formed of a polymer chain of amino-group-containing units and has a molecular weight in a range of 1000 to 20,000; wherein
a mixture of the first and second liquids at a time of mixing them has pH in a range of 5.0 to 8.0
wherein the aldehyde-groups-introduced alpha-glucan is produced by oxidation of dextran having weight-average molecular weight in a range of 2000 to 100,000 or by oxidation of dextrin, with periodic acid or periodate, as to have aldehyde groups at a density of 0.1 to 1.0 per anhydroglucose unit, and
wherein the concentration of the alpha-glucan aldehyde in the first liquid is in a range of 5 to 50 wt% and the concentration of the amino-groups-containing polymer in the second liquid is in a range of 0.5 to 60 wt%.

2. Medical-use two-component adhesive according to claim 1, wherein molar ratio of aldehyde groups to amino groups in the mixture of the first and second liquids at a time of mixing them is in a range of 0.2 to 2.0.

3. Medical-use two-component adhesive according to claim 1, wherein the second liquid is added with; acetic acid, citric acid, succinic acid, glutar acid, malic acid, fumaric acid, maleic acid, or other monocarboxylic acid or polycarboxylic acid, or anhydride corresponding at least one of these acids; by 1 to 10wt%.

4. Medical-use two-component adhesive according to claim 1 or 2, wherein the first and second liquids have been subjected to sterilization with electron radiation in a range of 10 to 50KGy.

5. Medical-use two-component adhesive according to claim 1 or 2, wherein the amino-groups-containing polymer is epsilon-poly-L-lysine produced by microorganism or by enzyme.

6. Medical-use resin in a form of hydrogel that is formed by mixing the first and second liquids of the medical-use two-component adhesive according to any one of claims 1 to 5, wherein reaction molar ratio of aldehyde groups to amino groups is in a range of 0.2 to 2.0.

## Patentansprüche

1. Zweikomponentenklebstoff für medizinische Verwendung, welcher umfasst: eine erste Flüssigkeit, die eine flüssige Lösung von alpha-Glucan mit eingeführten Aldehydgruppen und einem Gewichtsmittel des Molekulargewichts im Bereich von 1000 bis 200.000 umfasst, und eine zweite Flüssigkeit, die eine wässrige Lösung von Aminogruppen enthaltendem Polymer umfasst, das mindestens ein Polymer ist, das aus der Gruppe ausgewählt ist, die aus epsilon-Poly-L-lysin, alpha-Poly-L-lysin, Chitosan Oligomer und abgebautem Chitosan besteht, aus einer Polymerkette von Amingruppen enthaltenden Einheiten gebildet und ein Molekulargewicht im Bereich von 1000 bis 20.000 aufweist;
worin
ein Gemisch der ersten und zweiten Flüssigkeiten zum Zeitpunkt des Vermischens der beiden einen pH-Wert im Bereich von 5,0 bis 8,0 aufweist,
wobei das alpha-Glucan mit eingeführten Aldehydgruppen durch Oxidation von Dextran eines Gewichtsmittels des Molekulargewichts im Bereich von 2000 bis 100.000 oder durch Oxidation von Dextrin mit Periodsäure oder Periodat hergestellt wird, so dass es Aldehydgruppen in einer Dichte von 0,1 bis 1,0 pro Anhydroglucoseeinheit aufweist, und wobei die Konzentration des alpha-Glucanaldehyds in der ersten Flüssigkeit im Bereich von 5 bis 50 Gewichtsprozent und die Konzentration des Amingruppen enthaltenden Polymers in der zweiten Flüssigkeit im Bereich von 0,5 bis 60 Gewichtsprozent liegt.

2. Zweikomponentenklebstoff für medizinische Verwendung nach Anspruch 1, wobei das Molverhältnis der Aldehydgruppen zu Amingruppen in dem Gemisch der ersten und zweiten Flüssigkeiten zum Zeitpunkt des Vermischens der beiden im Bereich von 0,2 bis 2,0 liegt.

3. Zweikomponentenklebstoff für medizinische Verwendung nach Anspruch 1, wobei die zweite Flüssigkeit mit Essigsäure, Zitronensäure, Bernsteinsäure, Glutarsäure, Äpfelsäure, Fumarsäure, Maleinsäure oder einer anderen Monocarbonsäure oder Polycarbonsäure oder einem Anhydrid, das mindestens einer dieser Säuren entspricht, zu 1 bis 10 Gewichtsprozent versetzt ist.

4. Zweikomponentenklebstoff für medizinische Verwendung nach Anspruch 1 oder 2, wobei die ersten und zweiten Flüssigkeiten einer Sterilisation mit Elektronenstrahlen im Bereich von 10 bis 50 Kgy unterworfen worden sind.

5. Zweikomponentenklebstoff für medizinische Verwendung nach Anspruch 1 oder 2, wobei das Amingruppen enthaltende Polymer durch einen Mikroorganismus oder durch ein Enzym hergestelltes epsilon-Poly-L-lysin ist.

6. Harz für die medizinische Verwendung in der Form eines Hydrogels, das durch Mischen der ersten und zweiten Flüssigkeiten des Zweikomponentenklebstoffs für medizinische Verwendung nach einem der Ansprüche 1 bis 5 gebildet ist, wobei das Reaktionsmolverhältnis von Aldehydgruppen zu Amingruppen im Bereich von 0,2 bis 2,0 liegt.

## Revendications

1. Adhésif à deux composants destiné à un usage médical comprenant : un premier liquide constitué d'une solution aqueuse d'alpha-glucane dans lequel sont introduits des groupes aldéhyde, ayant un poids moléculaire moyen en poids dans une plage de 1 000 à 200 000, et un deuxième liquide constitué d'une solution aqueuse de polymère contenant des groupes amino qui est au moins un composé choisi dans un groupe constitué d'epsilon-poly-L-lysine, d'alpha-poly-L-lysine, d'un oligomère de chitosane, et de chitosane dégradé, constitué d'une chaîne polymère d'unités contenant des groupes amino et qui a un poids moléculaire dans une plage de 1 000 à 20 000 ;
dans lequel
un mélange des premier et deuxième liquides au moment de leur mélange a un pH dans une plage de 5,0 à 8,0,
dans lequel l'alpha-glucane dans lequel sont introduits des groupes aldéhyde est produit par oxydation de dextrane ayant un poids moléculaire moyen en poids dans une plage de 2 000 à 100 000 ou par oxydation de dextrine, avec de l'acide périodique ou du périodate, de manière à obtenir des groupes aldéhyde à une densité de 0,1 à 1,0 par unité anhydroglucose, et dans lequel la concentration de l'alpha-glucane-aldéhyde dans le premier liquide est dans une plage de 5 à 50 % en poids et la concentration du polymère contenant des groupes amino dans le deuxième liquide est dans une plage de 0,5 à 60 % en poids.

2. Adhésif à deux composants destiné à un usage médical selon la revendication 1, dans lequel le rapport molaire des groupes aldéhyde aux groupes amino dans le mélange des premier et deuxième liquides au moment de leur mélange est dans une plage de 0,2 à 2,0.

3. Adhésif à deux composants destiné à un usage médical selon la revendication 1, dans lequel le deuxième liquide est ajouté avec : de l'acide acétique, de l'acide citrique, de l'acide succinique, de l'acide glutarique, de l'acide malique, de l'acide fumarique, de l'acide maléique ou autre acide monocarboxylique ou acide polycarboxylique, ou un anhydride correspondant à au moins l'un de ces acides ; à raison de 1 à 10 % en poids.

4. Adhésif à deux composants destiné à un usage médical selon la revendication 1 ou 2, dans lequel les premier et deuxième liquides ont été soumis à une stérilisation par rayonnement électronique dans une plage de 10 à 50 KGy.

5. Adhésif à deux composants destiné à un usage médical selon la revendication 1 ou 2, dans lequel le polymère contenant des groupes amino est l'epsilon-poly-L-lysine produite par un microorganisme ou par une enzyme.

6. Résine destinée à un usage médical sous la forme d'un hydrogel qui est formé en mélangeant les premier et deuxième liquides de l'adhésif à deux composants destiné à un usage médical selon l'une quelconque des revendications 1 à 5, dans lequel le rapport molaire réactionnel des groupes aldéhyde aux groupes amino est dans une plage de 0,2 à 2,0.
